# FASCICULE DE BREVET EUROPEEN

(11) **EP 2 231 074 B1**
(45) Date de publication et mention de la délivrance du brevet: **07.03.2012**
(21) Numéro de dépôt: 09703294.0
(22) Date de dépôt: 14.01.2009
(51) Int. Cl.: A61F 2/44, A61F 2/46

(54) **ENSEMBLE DE STABILISATION INTERVERTEBRALE POUR ARTHRODESE COMPRENANT UN CORPS DE CAGE A IMPACTION, ET SON ANCILLAIRE DE POSE**
WIRBELSÄULENSTABILISIERUNGSANORDNUNG FÜR ARTHRODESE MIT EINEM EINSCHLAGSKÄFIGELEMENT UND HILFSVORRICHTUNG ZU IHRER IMPLANTIERUNG
INTERVERTEBRAL STABILIZATION ASSEMBLY FOR ARTHRODESIS, COMPRISING AN IMPACTION CAGE BODY, AND AN ANCILLARY DEVICE FOR IMPLANTING SAME

(30) Priorité: 15.01.2008 FR 0850220; 03.04.2008 FR 0852208; 30.09.2008 FR 0856560
(43) Date de publication de la demande: 29.09.2010
(73) Titulaire: Graf, Henry, 69006 Lyon (FR)
(72) Inventeur: Graf, Henry, 69006 Lyon (FR)
(74) Mandataire: Myon, Gérard Jean-Pierre
(86) Numéro de dépôt international: PCT/FR2009/050048
(87) Numéro de publication internationale: WO 2009/092961

(56) Documents cités:
- EP-A- 0 646 366
- WO-A-99/60957
- WO-A-2005/112834
- WO-A-2006/051547
- WO-A-2007/090017
- DE-A1- 10 035 182
- US-A- 5 803 904
- US-A1- 2005 131 405
- US-B1- 6 830 570

## Description

La présente invention concerne un ensemble de stabilisation intervertébrale comprenant un corps de cage à impaction, ainsi qu'un ancillaire de pose de cet ensemble de stabilisation.

L'invention se situe dans le domaine de l'arthrodèse, à savoir de la fusion osseuse entre au moins deux vertèbres adjacentes. On rappellera que l'arthrodèse vise à autoriser uniquement des micro-mouvements entre les vertèbres, ainsi qu'un amortissement des vibrations. Ces micros-mouvements permettent entre autres au patient, redevenu bipède après l'intervention, d'adapter son équilibre au mieux avant la prise de la greffe osseuse.

De manière typique, un ensemble extra-discal pour arthrodèse, au sens de l'invention, autorise une amplitude de mouvements entre deux vertèbres, qui est égale au plus à environ 10% de l'amplitude physiologique naturelle. En d'autres termes, s'il existe une amplitude naturelle maximale de 10° en rotation entre deux vertèbres données, l'ensemble de stabilisation conforme à l'invention est susceptible d'autoriser, au plus, un débattement de 1° entre ces deux vertèbres. Cette plage de mouvement autorisée s'établit autour d'un positionnement choisi, déterminé par le chirurgien lors de l'opération, notamment en fonction de la pathologie. On notera également que ce positionnement choisi, s'il appartient bien sûr à la plage de mouvement autorisé, ne se situe pas nécessairement au milieu de cette dernière.

De manière habituelle, une cage intervertébrale est destinée à remplacer tout ou partie d'un disque, lorsque ce dernier a été détruit par la chirurgie ou par la maladie. Le rôle principal d'une telle cage est de restaurer la hauteur discale, à savoir l'écartement entre les deux corps vertébraux en regard. Elle assure également une fonction de stabilisation antérieure, en permettant la création d'une fusion osseuse, dans le cas de l'arthrodèse.

L'invention vise plus particulièrement un ensemble de stabilisation, qui comprend un corps de cage pouvant être introduit par impaction dans l'espace intervertébral. La méthode de pose classique, associée à ce type de cage, fait tout d'abord appel à des bougies de dilatation vertébrale de taille croissante, dont les dimensions varient typiquement de 8 à 13 mm.

La dilatation de l'espace intervertébral est obtenue par rotation d'un quart de tour, de sorte que la bougie se retrouve placée sur la tranche en écartant mutuellement les vertèbres selon une distance correspondant à la largeur de cette bougie. Une fois que le volume nécessaire à l'introduction de la cage a été créé, on impacte cette dernière dans l'espace intervertébral.

Les cages classiques présentent des inconvénients, liés en particulier à leur méthode de pose. En effet, ces méthodes se révèlent peu commodes à mettre en oeuvre, voire dangereuses pour le patient, dans la mesure où la manipulation de la racine nerveuse s'opère de façon assez brutale. En outre, plus la cage est haute, plus cette manoeuvre d'impaction est difficile à réaliser. Un ensemble de stabilisation selon le préambule de la revendication 1 est decrit dans WO 99/60957.

L'invention vise à remédier à ces différents inconvénients. Elle vise en particulier à proposer un ensemble de stabilisation intervertébrale dont la structure permet d'implanter, de manière simple et moins dangereuse pour le patient, un corps de cage dans l'espace intervertébral.

A cet effet, elle a pour objet un ensemble de stabilisation intervertébrale pour arthrodèse, comprenant un corps de cage, destiné à être introduit par impaction dans l'espace intervertébral, cet ensemble comprenant en outre un nez d'introduction du corps de cage dans l'espace intervertébral, ce nez étant solidaire ou propre à être solidarisé avec ce corps de cage, dans une position excentrée, de sorte que, quand on introduit ce nez dans l'espace intervertébral, la mise en rotation de ce nez induit une ouverture de cet espace intervertébral et, de façon simultanée, un déplacement du corps de cage à la façon d'une came.

Une méthode de pose de l'ensemble ci-dessus, comprenant les étapes suivantes :
- on introduit le nez dans l'espace intervertébral par sa plus petite dimension, de façon à donner une première hauteur à cet espace intervertébral, tout en laissant le corps de cage à l'extérieur de cet espace intervertébral ;
- on tourne le nez autour de son axe principal, tout en laissant le corps de cage à l'extérieur de l'espace intervertébral, de manière à augmenter la hauteur de cet espace intervertébral ; et
- on introduit axialement le corps de cage dans l'espace intervertébral ainsi rehaussé.

Selon d'autres caractéristiques :
- on introduit le nez sur le côté de l'espace intervertébral, en référence à l'axe sagittal médian, en disposant la partie du corps de cage opposée au nez, de sorte qu'elle fasse saillie vers le centre de la colonne lors de la mise en rotation du nez, de manière à écarter la racine nerveuse ;
- on tourne le nez, de manière à augmenter la hauteur de l'espace intervertébral, puis on introduit axialement le corps de cage ;
- on tourne le nez, en même temps qu'on introduit axialement le corps de cage ;
- on immobilise le nez par rapport au corps de cage avant d'introduire ce nez dans l'espace intervertébral et, après avoir mis en place cette cage dans l'espace intervertébral, on désolidarise le nez par rapport au corps de cage ;
- on désolidarise le nez, en dévissant la tige filetée de l'organe de préhension par rapport à l'embase taraudée du nez ;
- on solidarise le corps de cage par rapport à l'organe de pose, de sorte que cet organe de préhension forme le nez d'introduction, puis on désolidarise cet organe de préhension après mise en place de la cage ;
- afin de désolidariser l'organe par rapport au corps de cage, on repousse ce corps de cage au moyen du piston, de façon à ressortir les branches hors des rainures ;
- on place le premier insert dans l'enveloppe du corps de cage, de manière à former le nez d'introduction puis, après introduction du corps de cage dans l'espace intervertébral, on enlève ce premier insert hors du volume intérieur de l'enveloppe, et on introduit éventuellement au moins un insert supplémentaire dans cette enveloppe.

L'invention va être décrite ci-après, en référence aux dessins annexés, donnés uniquement à titre d'exemples non limitatifs, dans lesquels :
- les figures 1 et 2, ainsi que 3 à 5, sont des vues respectivement en perspective, de côté, de devant et de dessus, illustrant un ensemble de stabilisation conforme à l'invention ;
- la figure 6 est une vue en perspective, illustrant un ancillaire de pose de l'ensemble de stabilisation des figures 1 à 5 ;
- les figures 7 à 9 sont des vues de derrière, illustrant la mise en place de l'ensemble de stabilisation des figures 1 à 5, au moyen de l'ancillaire de la figure 6 ;
- la figure 10 est une vue en perspective, analogue à la figure 6, illustrant une première variante de réalisation de l'invention,
- la figure 11 est une vue en perspective, analogue à la figure 2, illustrant une autre variante de réalisation de l'invention ;
- les figures 12 et 13 sont des vues respectivement de côté et en perspective, illustrant une autre variante de réalisation de l'invention ;
- les figures 14 à 17 sont des vues respectivement de côté et en perspective, illustrant encore une autre variante de réalisation de l'invention ;
- les figures 18 et 19 sont des vues de derrière, analogues aux figures 6 à 8, illustrant la mise en place de l'ensemble de stabilisation des figures 14à17;
- les figures 20 et 21 sont des vues en perspective, illustrant la mise en oeuvre d'une autre variante de réalisation de l'invention ;
- les figures 22 à 24 sont des vues respectives de dessus, de côté et en perspective, illustrant une variante supplémentaire de réalisation de l'invention ;
- la figure 25 est une vue de derrière, illustrant un corps de cage intervenant dans le mode de réalisation des figures 22 à 24 ;
- les figures 26 et 27 sont des vues respectivement de dessus et de derrière, illustrant une autre variante de réalisation de l'invention ; et
- les figures 28 et 29 sont des vues respectivement de derrière et en perspective, illustrant un mode de réalisation qui n'appartient pas à l'invention ;
- les figures 30 à 32 sont des vues en coupe longitudinale, illustrant trois étapes de la mise en oeuvre d'un mode supplémentaire de l'invention ;
- les figures 33 et 34 sont des vues en coupe longitudinale, illustrant deux étapes de la mise en ceuvre d'un autre mode de réalisation de l'invention ;
- les figures 35 à 38 sont des vues en perspective, illustrant quatre étapes de la mise en oeuvre d'une variante supplémentaire de l'invention ;
- les figures 39 et 40 sont des vues en perspective, illustrant deux étapes de la mise en oeuvre d'un exemple supplémentaire de réalisation de l'invention ;
- les figures 41 et 42 sont des vues en coupe longitudinale, illustrant deux étapes supplémentaires de mise en oeuvre du mode de réalisation des figures 39 et 40 ;
- les figures 43 et 44 sont des vues respectivement en coupe longitudinale et en bout d'un mode de réalisation supplémentaire de l'invention ;
- les figures 45 à 47 sont des vues respectivement en perspective et de face, illustrant encore un autre mode de réalisation de l'invention ;
- les figures 48 et 49 sont deux vues en perspective illustrant la mise en oeuvre d'un autre mode de réalisation de l'invention ;
- les figures 50 à 53 sont des vues de face, avec coupe partielle, illustrant quatre autres modes de réalisation, seul celui de la figure 51 appartenant à l'invention.

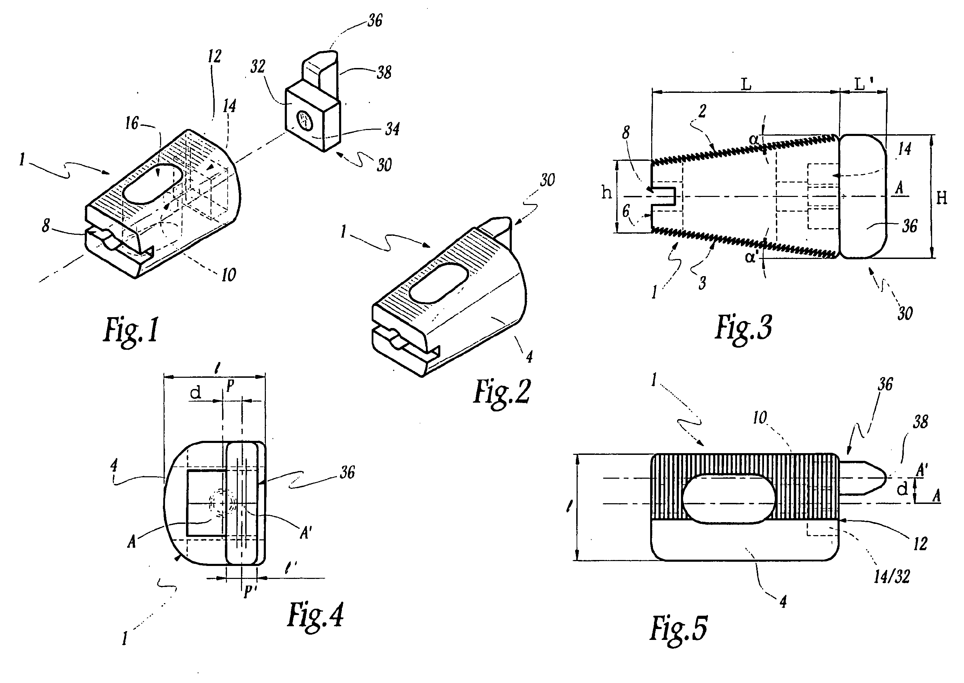

Les figures 1 à 8 illustrent un premier mode de réalisation, dans lequel l'ensemble de stabilisation conforme à l'invention comprend un corps de cage, désigné dans son ensemble par la référence 1, ainsi qu'un insert amovible 30, qui comprend notamment un nez. Dans ce qui suit, les termes relatifs au positionnement, tels que notamment inférieur, supérieur, avant et arrière, sont relatifs à un corps de cage implanté entre deux vertèbres d'un patient, lui-même en position debout. Ainsi, sur les figures 3, 4 et 5, le corps de cage est représenté respectivement de côté, depuis l'avant, et de dessus.

Le corps de cage 1 représente une forme sensiblement parallélépipédique. Vu de côté, il possède une hauteur qui augmente sensiblement de façon continue en direction de l'avant. On note ainsi h et H les hauteurs extrêmes, à savoir respectivement arrière et avant de ce corps de cage 1 (à gauche et à droite sur la figure 3). On note également L et I la longueur et la largeur de ce corps de cage, qui sont sensiblement constantes.

On note en outre A l'axe longitudinal médian du corps de cage, qui s'étend d'arrière en avant. On note enfin α et α' les angles dits de mise en lordose, qui sont formés entre l'axe principal A et les surfaces respectivement supérieure 2 et inférieure 3 du corps de cage.

En ce qui concerne les de valeurs numériques, à titre purement indicatif, la hauteur h est comprise entre 5 et 8 mm, la hauteur H est comprise entre 7 et 15 mm, la longueur L est comprise entre 20 et 25 mm, alors que la largeur I est comprise entre 12 et 14 mm. De plus, les angles α et α' sont avantageusement égaux, et présentent une valeur comprise entre 4 et 8°. Dans ces conditions, l'angle global de mise en lordose, correspondant à la valeur (α + α'), est comprise entre 8 et 16°, notamment voisine de 15° .

Le corps de cage 1, de forme parallélépipédique, est tronqué par un bord latéral arrondi 4, dépourvu d'arêtes vives, dont la fonction sera décrite dans ce qui suit. De plus, la face arrière 6 du corps 1 est creusée d'une entaille transversale 8 qui communique avec une ouverture axiale 10 de passage d'un outil. Enfin, au niveau de la face avant 12, cette ouverture 10 débouche dans un logement 14, de forme parallélépipédique, dont les parois sont carrées, en section transversale.

Les surfaces supérieure 2 et inférieure 3, évoquées ci-dessus, présentent une surface crantée, afin d'assurer de façon classique une meilleure accroche contre les plateaux vertébraux. Une échancrure 16 est en outre creusée dans le corps de cage 1, en reliant ces surfaces supérieure et inférieure. Cette échancrure permet le passage d'un greffon osseux, de manière également connu en soi.

L'ensemble de stabilisation conforme à l'invention comprend en outre l'insert 30 précité, qui comporte une embase parallélépipédique 32, dont la forme est complémentaire de celle du logement 14. Cette embase 32 est creusée d'un taraudage axial 34, qui s'étend dans le prolongement de l'ouverture 10.

L'embase 32 supporte en outre un nez 36, en saillie vers l'avant du corps de cage. Vu de côté, sur la figure 3, ce nez forme le prolongement de ce corps de cage, à savoir que sa hauteur est égale à la hauteur maximale H du corps de cage. En revanche, vu de devant et de dessus, aux figures 4 et 5, ce nez est excentré par rapport au corps de cage.

De façon plus précise, si l'on note P le plan vertical médian du corps de cage, qui contient l'axe A, le nez 36 se trouve placé à l'opposé de ce plan médian, par rapport au bord latéral 4. On note ainsi l'axe A' longitudinal du nez, en vue de dessus à la figure 5, ainsi que P' le plan vertical médian du nez, en vue de devant à la figure 4. On note que A' et P' sont décalés, respectivement par rapport à l'axe principal A et au plan principal P du corps de cage 1.

Sur ces figures 4 et 5, on note d de la valeur du décalage précité. De façon avantageuse, d est compris entre 15 et 30% de la largeur du corps de cage. De plus, si l'on note I' la largeur du nez, cette largeur I' est nettement inférieure à la largeur I du corps de cage alors que, comme vu précédemment, la hauteur de ce nez correspond à la plus grande hauteur H du corps de cage. Dans ces conditions, le nez 36 présente une petite dimension, correspondant à sa largeur, ainsi qu'une grande dimension correspondant à sa hauteur.

On note par ailleurs L' la longueur du nez, à savoir la distance selon laquelle ce nez fait saillie vers l'avant, depuis la face avant du corps de cage 1. De façon avantageuse, cette longueur L' est comprise entre 15 et 25% de la longueur totale L du corps de cage. Enfin, le nez 36 a une pointe terminale effilée 38, permettant une meilleure introduction de ce nez entre les vertèbres adjacentes.

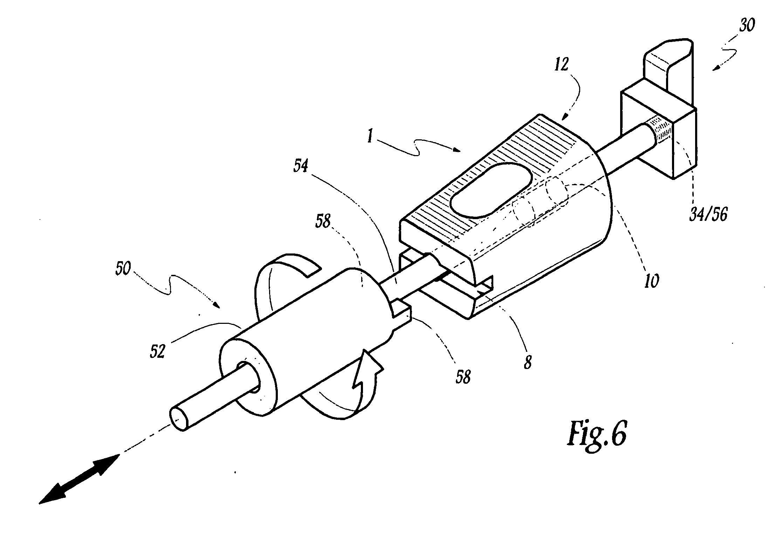

La figure 6 illustre un ancillaire 50, permettant la pose de l'ensemble de stabilisation des figures précédentes, formé du corps de cage 1 et de l'insert 30. Cet ancillaire 50 comprend un organe de préhension 52, qui supporte une tige 54, montée coulissante, qui est propre à pénétrer dans l'ouverture 10 du corps de cage 1. Cette tige 54 présente une extrémité filetée 56, propre à coopérer avec le taraudage 34 de l'insert 30. De plus, de part et d'autre de cette tige 54, l'organe de préhension 52 est solidaire de deux nervures 58, propres à être encastrées dans l'entaille 8 du corps de cage 1.

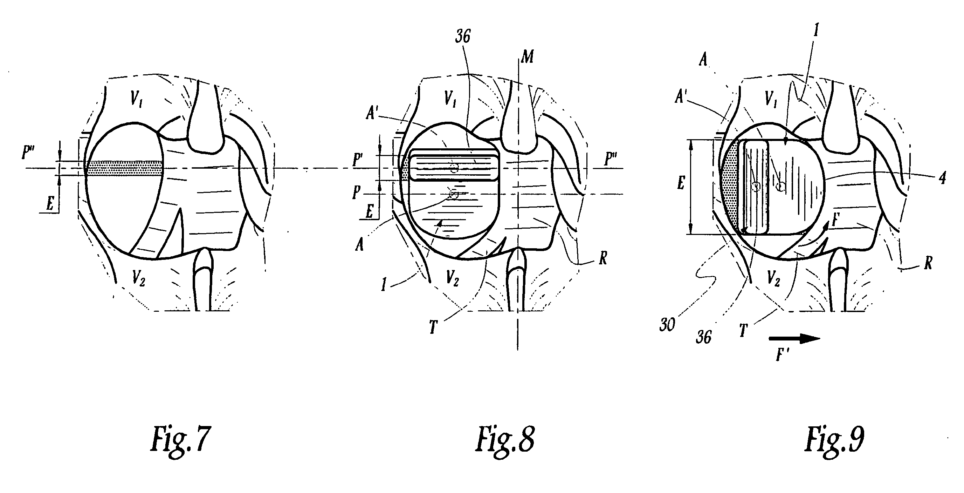

En vue de la pose du corps de cage 1 et de son insert 30, on prépare tout d'abord l'espace intervertébral, illustré de manière schématique sur la figure 7. On note V₁ et V₂ les deux vertèbres adjacentes, ainsi que E l'espace intervertébral proprement dit. Cette préparation est mise en oeuvre au moyen de bougies de dilatation de type classique, non représentées. De plus, on procède à un avivement des plateaux vertébraux, également de manière habituelle.

Par ailleurs, on immobilise l'insert 30 par rapport au corps 1, tout en bloquant mutuellement ce corps 1 et l'ancillaire 50. A cet effet, on place l'embase 32 dans le logement 14, et on visse l'extrémité filetée 56 de la tige 54 dans le taraudage 34. De plus, on verrouille les deux nervures 58 dans l'entaille 8. Dans ces conditions, le corps de cage 1, l'insert 30 et l'ancillaire 50 sont solidarisés les uns aux autres.

On introduit alors le nez 36 dans l'espace intervertébral, par sa plus petite dimension, à savoir par sa largeur. Comme le montre la figure 8, cette introduction est opérée sur l'un des côtés de l'espace intervertébral, en l'occurrence le côté gauche, à savoir à distance de l'axe médian M de la colonne vertébrale. On note en outre R la racine principale, ainsi que T les terminaisons latérales nerveuses.

Soit P" le plan horizontal médian de l'espace intervertébral. Une fois que le nez est introduit par sa plus petite dimension, ce plan P" coïncide avec le plan principal P' du nez, alors que le plan principal P du corps de cage 1 se situe de façon décalée par rapport au plan médian P". De façon générale, ce plan P se situe habituellement au-dessous des deux plans P' et P".

Cependant, s'il n'existe qu'un faible décalage entre le plan principal P du corps 1 et le plan principal P' du nez, il est alors possible de disposer la plus grande partie du corps de cage au-dessus du plan médian de l'espace intervertébral. En revanche, dans le cas d'un décalage plus important, on retiendra préférentiellement l'option illustrée sur la figure 8, sur laquelle la plus grande partie du corps de cage se situe au-dessous de l'espace intervertébral à savoir en regard de la vertèbre inférieure V₂. De plus, l'axe principal A' du nez passe par l'espace intervertébral E, alors que l'axe principal A du corps de cage passe par cette vertèbre inférieure.

Le chirurgien agit alors sur l'organe de préhension 52, en lui appliquant une rotation selon le sens anti-horaire matérialisé par la flèche F (voir figure 9). Etant donné que le nez est enfoncé dans l'espace intervertébral, cette rotation s'effectue selon l'axe principal A' du nez. Or, puisque le corps de cage n'est pas symétrique par rapport à cet axe principal, cette rotation s'accompagne d'un effet de came.

En d'autres termes, cette rotation induit le déplacement du corps de cage 1 vers le centre de la colonne vertébrale. De la sorte, le bord arrondi 4 vient en contact avec les racines R et les terminaisons T, ce qui tend à les repousser vers le centre de la colonne, selon la flèche F'. Ce déplacement des nerfs s'opère de manière douce, étant donné que le bord latéral 4 ne présente pas d'arête vive et que cette mise en mouvement s'exerce de façon progressive.

Comme illustré à la figure 9, au terme de la rotation décrite ci-dessus, qui s'opère selon environ ¼ de tour, le nez 36 pénètre dans l'espace intervertébral E par sa hauteur H, à savoir sa plus grande dimension. Cet espace intervertébral se trouve donc fortement rehaussé, de sorte que l'introduction axiale du corps de cage est grandement facilitée.

Sur les figures 8 et 9, le corps de cage est placé à gauche de l'axe médian, et on lui applique une rotation selon le sens anti-horaire. Dans le cas où le corps de cage est situé à droite, il s'agit bien entendu de le mettre en rotation selon le sens horaire, afin que son bord arrondi repousse les racines nervures.

Enfin, après avoir fait pivoter le nez, qui se trouve désormais dans sa position de la figure 9, on enfonce axialement le corps de cage 1 à l'intérieur de l'espace intervertébral E. Lorsque le corps de cage est mis en place, ses surfaces respectivement supérieure 2 et inférieure 3 prennent appui contre le plateau des vertèbres en regard V₁ et V₂. De plus, le bord latéral 4 se trouve tourné vers le centre de la colonne vertébrale.

Une fois le corps de cage introduit, on dévisse la tige 54 par rapport au taraudage 34, de sorte que l'insert 30 supportant le nez 36 n'est plus solidarisé par rapport au corps de cage 1. Il est donc possible de repousser cet insert grâce à un poussoir adapté, non représenté. Cet insert 30 retombe alors sur le plateau de la vertèbre inférieure V2.

De façon avantageuse, l'insert 30 est réalisé en un matériau biocompatible, susceptible de former un greffon osseux. Dans ces conditions, il s'agit par exemple de PEEK ou de BMP (Bone Morphogenic Protein). Enfin, après avoir désolidarisé l'insert 30 par rapport au corps de cage 1, on retire les nervures 58 hors de l'entaille 8, de manière à libérer l'ancillaire 50 par rapport à ce corps de cage, mis en place dans l'espace intervertébral.

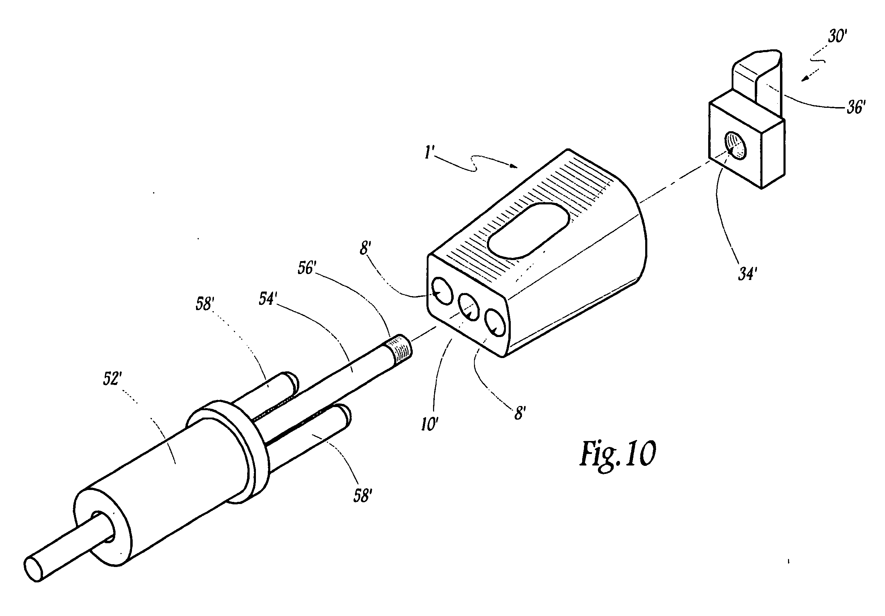

La figure 10 illustre une première variante de réalisation de l'invention, qui concerne plus spécifiquement le mode de solidarisation de l'ancillaire de pose par rapport au corps de cage. Sur cette figure 10, les éléments mécaniques analogues à ceux du premier mode de réalisation y sont associés aux mêmes numéros affectés de la référence « prime ».

Contrairement au premier mode de réalisation, on ne retrouve pas la coopération de deux nervures et d'une entaille transversale. En revanche, l'organe de préhension 52' est pourvu de deux doigts 58', qui pénètrent dans des trous borgnes 8', ménagés en regard dans le corps de cage 1'.

En vue de la solidarisation de l'ancillaire 50' et du corps de cage 1', on introduit les doigts 58' dans les trous borgnes 8', tout en vissant l'extrémité filetée 56' dans le taraudage 34'. L'implantation du corps de cage 1', au moyen du nez 36', s'opère de façon analogue à ce qui a été décrit en référence au premier mode de réalisation. Cette première variante est avantageuse, dans la mesure où elle assure une préhension satisfaisante de la cage au moment de l'effort en rotation.

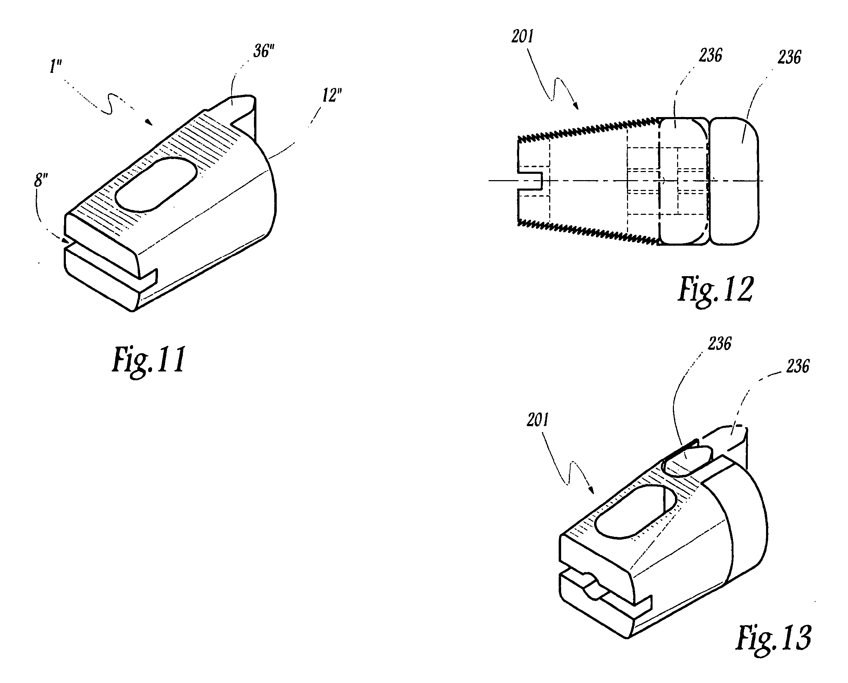

La figure 11 illustre une seconde variante de réalisation de l'invention. Sur cette figure 11, les éléments mécaniques analogues à ceux du premier mode de réalisation y sont associés aux mêmes numéros, affectés de la référence « seconde ».

Contrairement aux deux premiers modes de réalisation, le nez 36" est réalisé d'un seul tenant avec le corps de cage 1 ". En d'autres termes, ce corps et ce nez forment un ensemble monobloc. Par conséquent, le corps de cage est dépourvu de logement, tel celui 14 du premier mode de réalisation. Sa face avant 12" se prolonge directement par le nez 36", venu de matière.

Par ailleurs, l'ancillaire de pose, non représenté, est analogue à l'un de ceux décrits, en référence aux deux premiers modes de réalisation. Cependant, cet ancillaire est dépourvu de tige, telle que celle 54 de la figure 6. L'ancillaire peut être pourvu d'une nervure transversale unique, propre à pénétrer dans une entaille 8" du corps de cage. Cependant, on peut prévoir de munir cet ancillaire de doigts, tels ceux 58', propres à pénétrer dans deux trous borgnes, tels que ceux 8' de la figure 10.

Cette seconde variante de réalisation est avantageuse, notamment en termes de simplicité. En effet, cette cage présente une structure mécanique simple, qui s'accompagne d'une grande robustesse. De plus, elle nécessite un nombre de manipulations faible.

Selon une variante supplémentaire, le corps de cage peut être associé à un nez, qui ne se trouve pas rapporté de manière amovible, comme sur les figures 1 à 10, et qui n'est pas non plus monobloc comme sur la figure 11. Dans cette variante représentée aux figures 12 et 13, ce nez 236 est rétractable, à savoir qu'il est propre à occuper deux positions axiales différentes.

Dans la première de ces positions axiales, le nez fait saillie au-delà du corps de cage 201, de sorte qu'il est à même d'être introduit dans l'espace intervertébral, afin d'assurer l'effet came du corps de cage excentré. Ce nez est alors mis en rotation, comme expliqué précédemment, de manière à rehausser l'espace intervertébral, puis la cage est introduite axialement dans ce dernier. Enfin, ce nez est rétracté à l'intérieur du corps de cage, de sorte qu'il ne fait plus saillie vers l'avant de ce dernier. Ceci est avantageux, car ce nez ne présente alors aucun encombrement supplémentaire, au sein de l'espace intervertébral.

Dans la variante des figures 12 et 13, le blocage du nez, dans sa position axialement rétractée et dans sa position axialement avancée, est assuré par tout moyen approprié, notamment grâce à un rivet. De plus, on notera que, sur ces figures 12 et 13, le corps de cage présente une partie arrière tronconique, ainsi qu'une partie avant de hauteur constante, dans laquelle le nez est logé dans sa position rétractée. Cependant, ce nez rétractable peut être appliqué à une cage, dont la hauteur est constante, ou bien continûment variable, sur toute sa longueur.

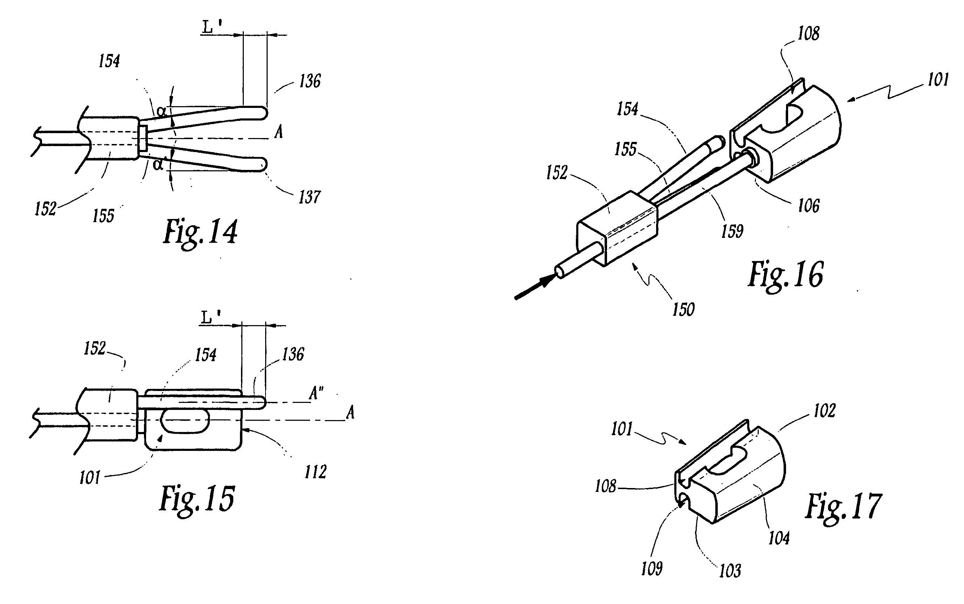

Les figures 14 et suivantes illustrent une troisième variante de réalisation. Sur cette figure, les éléments mécaniques analogues à ceux du premier mode de réalisation y sont affectés des mêmes numéros de référence, augmentés de 100.

Le corps de cage 101 diffère de celui 1 du premier mode de réalisation, notamment en ce qu'il est dépourvu d'entaille transversale. Cette dernière est remplacée par deux rainures longitudinales 108 et 109, creusées respectivement dans les surfaces supérieure 102 et inférieure 103.

En vue de dessus, ces deux rainures s'étendent selon un axe A", qui est décalé par rapport à l'axe principal A du corps de cage. Par comparaison avec la figure 5, cet axe A" correspond sensiblement à celui A' du nez 36. On notera par conséquent que ces rainures s'étendent de manière décalée, par rapport à l'axe et au plan médians du corps de cage.

De plus, par comparaison au premier mode de réalisation, le corps de cage 101 est dépourvu d'une ouverture axiale, telle que celle 10. On ne retrouve pas non plus de logement, tel que celui 14.

Le corps de cage 101 est associé à un organe de pose 150, qui présente un corps de préhension 152, qui se prolonge par deux tiges 154 et 155, propres à pénétrer dans les rainures 108 et 109. Vues de côté, ces deux branches s'étendent de manière divergente à partir du corps 152, selon les angles α et α' correspondant aux angles de mise en lordose du corps de cage, dont la hauteur augmente vers l'avant.

Chaque tige 154, 155 est terminée par un embout respectif 136, 137. Contrairement à ces branches, ces embouts 136 et 137 sont « droits », à savoir qu'ils sont parallèles à l'axe principal A, vus de côté. Enfin, le corps 152 est pourvu d'un piston 159, mobile entre une position escamotée et une position dans laquelle il fait saillie vers l'avant.

La solidarisation de l'organe de pose 150 et du corps de cage 101 s'opère de la manière suivante. Il' s'agit tout d'abord d'introduire les branches 154 et 155 dans les rainures 108 et 109. Au terme de cette mise en place, les embouts 136 et 137 font saillie, depuis la face avant 112 du corps de cage 101, selon une distance L' correspondant sensiblement à la longueur du nez du premier mode de réalisation.

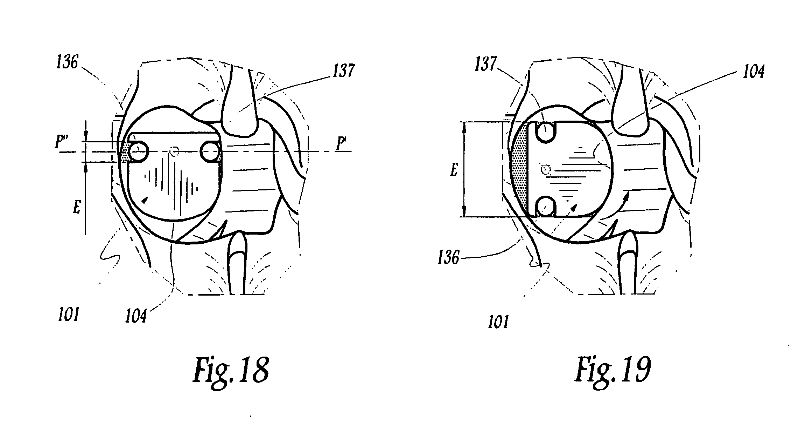

Comme le montre la figure 18, on introduit ensuite les deux embouts 136 et 137 dans l'espace intervertébral, de façon analogue à la mise en place du nez 36, notamment en référence à la figure 7. Au terme de cette phase initiale d'introduction, les deux embouts sont disposés l'un à côté de l'autre, à savoir que l'espace intervertébral présente une faible hauteur.

Puis, on fait pivoter l'ensemble composé du corps de cage 101 et de l'organe de pose 150, dans le sens anti-horaire, comme illustré à la figure 19. On conçoit que, dans ce dernier mode de réalisation, les deux embouts 136 et 137 assurent la même fonction d'axe de rotation excentrée, que le nez 36 du premier mode de réalisation. Dans ces conditions, le bord arrondi 104 du corps de cage repousse les racines nerveuses, par effet de came, comme dans ce premier mode de réalisation.

Au terme d'une rotation d'un quart de tour, les embouts 136 et 137 sont désormais placés l'un au-dessus de l'autre, de sorte que l'espace intervertébral E est fortement rehaussé (figure 19). On peut alors introduire axialement le corps de cage dans l'espace intervertébral, comme décrit en référence au premier mode de réalisation. Une fois ce corps de cage mis en place, on actionne le piston 159, qui prend alors appui contre la face arrière 106. Ceci permet donc de retirer les branches 154 et 155 hors des rainures 108 et 109, de façon à désolidariser l'organe de pose 150 par rapport au corps de cage 101.

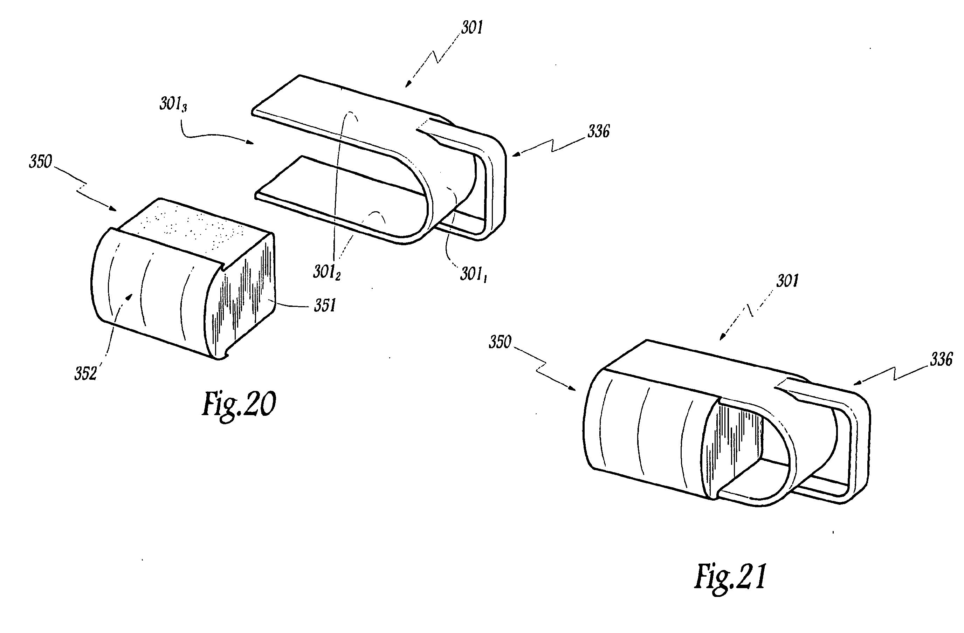

Sur les figures 20 et 21, on retrouve un corps de cage 301 qui présente une forme de C. Ce corps 301 possède ainsi une âme 301₁ destinée à être placée à l'avant de l'espace intervertébral, ainsi que deux ailes 301₂, dont chacune prend appui contre un corps vertébral respectif. Ce corps de cage 301, qui peut être assimilé à une lame ressort, possède une extrémité ouverte 301₃, tournée vers l'arrière du patient, qui est propre à être déformée de manière préférentielle lors de la mise en lordose.

Dans cet exemple, le corps de cage déformable 301 présente une forme de C. Cependant, on peut prévoir qu'il est conforme à l'un quelconque des modes de réalisation, décrits dans FR-A-2 913 328, au nom du présent Demandeur. Ainsi, le corps de cage peut notamment se présenter sous forme d'un ruban, définissant une boucle fermée.

En revenant aux figures 20 et 21, le corps de cage 301 est associé à un nez 336, qui peut par exemple être assimilé au nez 36" de la figure 11. Ainsi, ce nez 336 est excentré, à savoir qu'il est décalé latéralement par rapport à l'axe longitudinal médian du corps de cage 301. Ce nez, également en forme de C, est situé dans le prolongement du corps 301.

Sur ces figures 20 et 21, on retrouve également un bloc auxiliaire 350, formant cache, réalisé en un matériau sensiblement rigide tel que le PEEK. Ce bloc 350 présente une partie principale 351, propre à être insérée entre les deux ailes du corps 301, ainsi qu'un bord arrondi 352, faisant saillie latéralement par rapport à ce corps de cage.

Ainsi, en vue de la pose, il s'agit tout d'abord d'introduire le bloc entre les deux ailes du corps de cage, dans la position de la figure 21, de sorte que le bord 352 assure la même fonction que, par exemple, le bord arrondi 4 illustré notamment à la figure 5. En vue de la pose, on introduit le nez dans l'espace intervertébral, comme décrit notamment en référence à la figure 8, puis on fait pivoter ce nez et le corps de cage d'un quart de tour, comme décrit notamment en référence à la figure 9.

Après mise en place du corps de cage dans l'espace intervertébral, on retire le bloc auxiliaire grâce à tout moyen approprié. Dans ces conditions, ce corps de cage est alors susceptible, tout d'abord, de se déformer de manière à autoriser le positionnement choisi des vertèbres en lordose, notamment sous l'action d'un haubanage postérieur, puis d'assurer sa fonction d'arthrodèse telle que définie ci-dessus. De plus, la présence du bloc 350 permet de cacher les arêtes vives du C, ce qui préserve la racine nerveuse.

On notera également que le nez assure, outre sa fonction de came, une fonction supplémentaire de stabilisation mécanique du corps de cage déformable. Ainsi, l'exemple illustré, ce nez permet d'éviter l'effondrement du corps de cage, au voisinage de son âme.

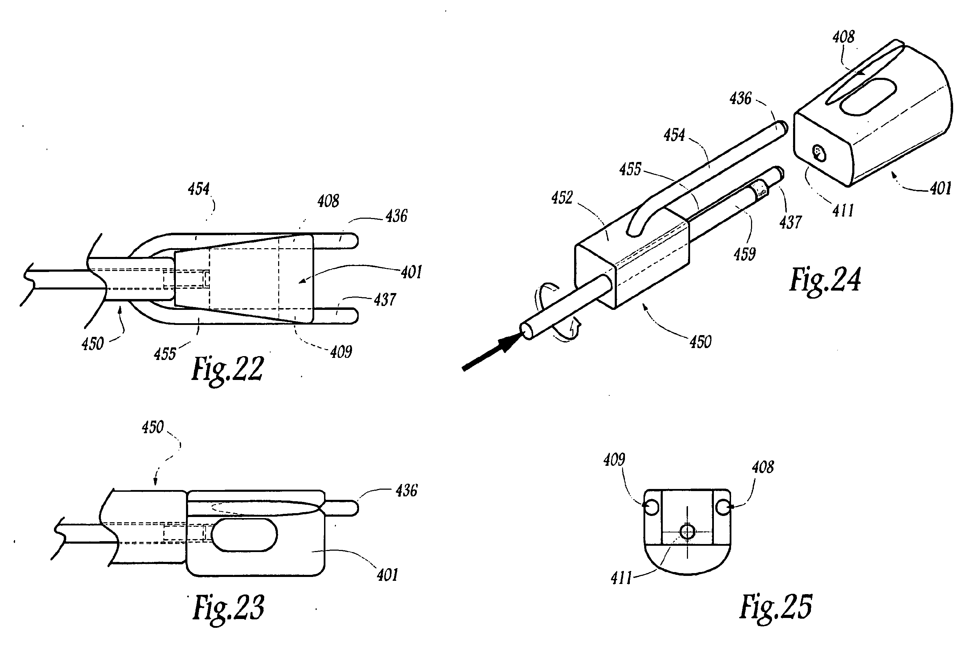

Un mode de réalisation supplémentaire, conforme à l'invention, est illustré sur les figures 22 à 25. Sur ces figures, les éléments mécaniques analogues à ceux des figures 14 à 17 y sont affectés des mêmes numéros de référence, augmentés de 300.

L'organe de pose 450 diffère de celui 150, en ce qu'il possède des tiges ou branches 454 et 455, qui sont terminées par des embouts 436 et 437 s'étendant dans le prolongement de ces tiges. En d'autres termes, non seulement les embouts, mais également les tiges sont parallèles à l'axe principal du corps de cage, vus de côté.

Ces tiges et ces embouts pénètrent dans des rainures 408 et 409, ménagées dans le corps de cage, qui s'étendent également de manière parallèle à l'axe principal A, du fait que les branches ne sont pas inclinées. Enfin, l'organe de pose 450 est muni d'une tige filetée 459, propre à pénétrer dans un trou taraudé 411, ménagé dans le corps de cage. Ceci permet d'assurer une prise et, par conséquent, une mise en place plus aisée du corps de cage.

Les différentes étapes de l'implantation du corps de cage sont analogues à celles décrites ci-dessus, en référence aux figures 14 à 17. On notera qu'il est avantageux de prévoir des tiges 454 et 455 parallèles à l'axe principal A. En effet, lorsqu'on les retire de l'espace intervertébral, ces branches sont particulièrement peu traumatisantes pour le patient, étant donné qu'elles ne présentent aucune aspérité.

L'invention n'est pas limitée aux exemples décrits et représentés.

Ainsi, dans la plupart des modes de réalisation présentés ci-dessus, le corps de cage présente une hauteur qui augmente vers l'avant. Cependant, à titre de variante, un nez excentré conforme à l'invention peut être associé à tout type de cage à impaction, dont la hauteur est invariante, voire augmente en direction, de sa partie arrière.

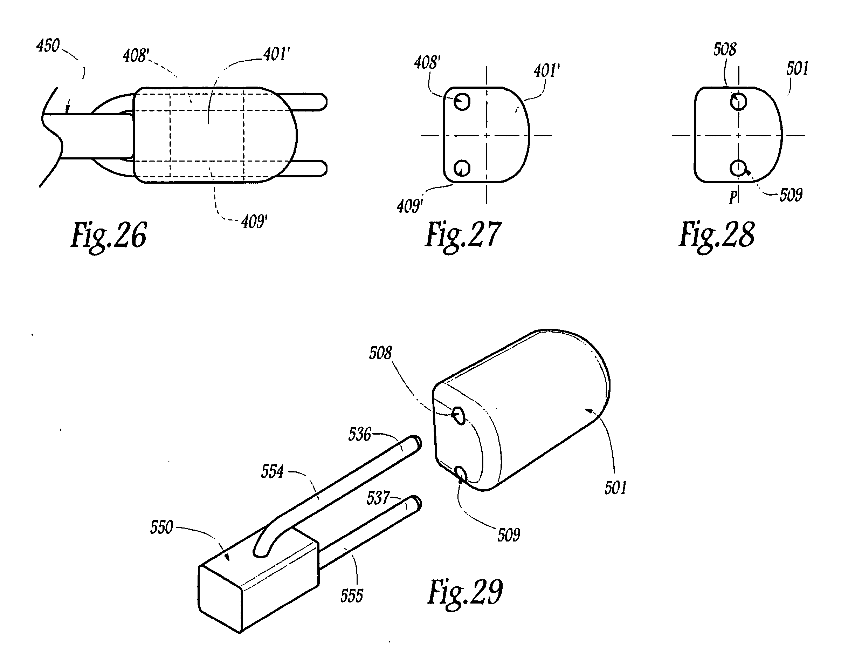

Un tel mode de réalisation est illustré sur les figures 26 et 27, qui sont à rapprocher des figures 22 et 25. En effet, sur ces différentes figures, on retrouve un même organe de pose 450, mais des corps de cage différents. Sur les figures 26 et 27, les éléments mécaniques de ce corps de cage, qui sont analogues à ceux des figures 22 à 25, possèdent les mêmes numéros affectés de la référence « prime ».

Comme le montre la figure 26, le corps de cage 401' présente une hauteur invariante, entre ses extrémités respectivement avant et arrière. Par conséquent, les rainures 408 et 409 sont remplacées par deux « tunnels », à savoir deux ouvertures 408' et 409' qui sont ménagées à l'intérieur du corps de cage. De plus, ces ouvertures 408' et 409', qui débouchent à la fois sur la partie avant et la partie arrière du corps de cage, ne débouchent cependant pas de manière latérale. A cet égard, on peut noter que ces ouvertures peuvent être réalisées sur un corps de cage préexistant, par tout moyen approprié tel une mèche. La pose d'un tel corps de cage 401', de hauteur invariante, est mise en oeuvre de la même façon que dans les modes de réalisation précédents.

On notera en outre que, dans les différents modes de réalisation ci-dessus, le nez est excentré par rapport au corps de cage, à savoir que leurs axes principaux sont distincts. Cependant, à titre de variante comme présenté aux figures 28 et 29, on peut prévoir qui le nez et le corps de cage sont coaxiaux. Dans ces conditions, il est possible d'adapter l'ensemble des caractéristiques techniques, décrites en référence à l'ensemble des figures de la présente demande, à un tel nez co-axial. Ce dernier assure alors principalement une introduction aisée du corps de cage, à l'intérieur de l'espace intervertébral.

Cette variante alternative est illustrée sur les figures 28 et 29, qui sont à rapprocher de celles 26 et 27. On retrouve un corps de cage 501, percé de deux ouvertures 508 et 509, qui sont situées dans le plan principal P, tel que défini à la figure 4. Ces ouvertures sont par conséquent « centrées », par opposition notamment à celles 408' et 409' illustrées sur la figure 27.

Ce corps de cage 501 coopère avec un organe de pose 550, qui est pourvu de deux branches 554 et 555, dont chacune est terminée par un embout respectif 536 et 537, s'étendant dans le prolongement de cette branche. Lorsqu'on enfile les branches précitées dans les ouvertures 508 et 509, les embouts 536 et 537 font saillie à l'opposé, à savoir à droite sur la figure 29. Ces deux embouts forment alors un nez de type centré, permettant l'introduction du corps de cage dans l'espace intervertébral.

L'invention permet d'atteindre les objectifs précédemment mentionnés.

En effet, le corps de cage conforme à l'invention assure, outre sa fonction classique d'écarteur intervertébral, une fonction supplémentaire d'écarteur de racine nerveuse. Ceci permet donc de simplifier la manoeuvre d'introduction, en la rendant moins dangereuse pour le tissu nerveux que dans l'art antérieur. De plus, grâce à l'invention, il est possible d'admettre à l'intérieur de l'espace intervertébral, des cages dont la hauteur est plus importante que dans l'art antérieur. En effet, de telles cages hautes ne pourraient être introduites de façon simple, selon les procédures employées dans l'état de la technique.

Enfin, l'invention permet de simplifier sensiblement la pose de la cage intervertébrale. En effet, le chirurgien est à même d'effectuer, en un seul geste, ce qui nécessite plusieurs mains dans l'état de la technique. Ceci permet en outre un contrôle amélioré, la réduction du nombre de manipulations aveugles, ainsi que plus de douceur dans la mise en place de la cage.

On va maintenant décrire différentes variantes de réalisation supplémentaires de l'invention. Pour chaque variante décrite, les éléments mécaniques analogues à ceux des figures précédentes sont augmentés de 100, par rapport à la variante de réalisation qui la précède immédiatement.

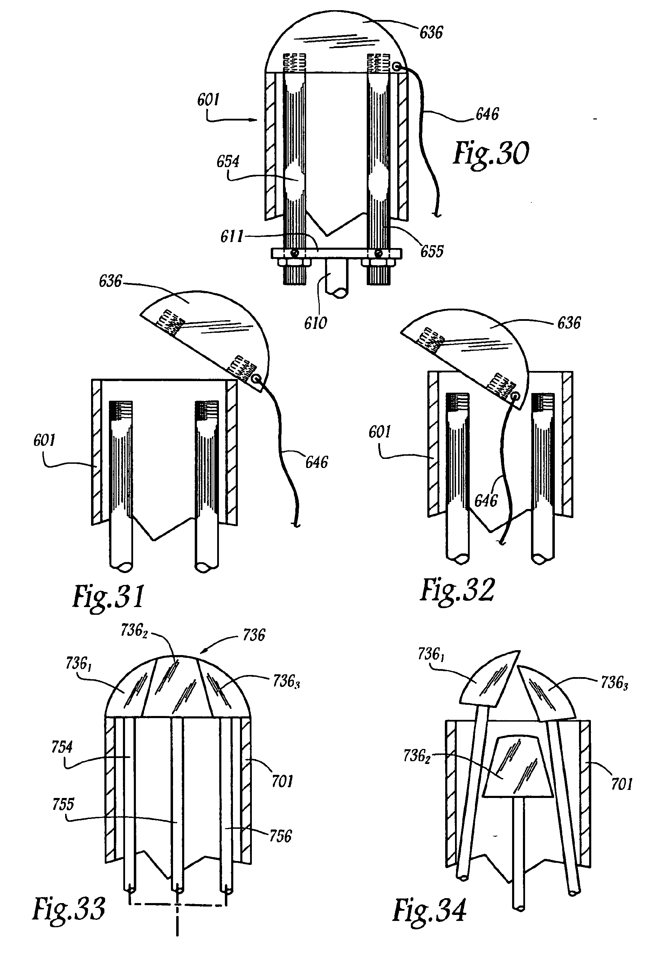

Sur les figures 30 à 32, on retrouve tout d'abord une variante de réalisation, dans laquelle les branches 654 et 655 ne forment pas de façon directe le nez comme, par exemple, dans le mode de réalisation des figures 22 et suivantes. Ces branches sont alors propres à coopérer avec un insert 636, formant nez d'introduction, lequel présente des dimensions plus faibles que, par exemple, l'insert 30 du premier mode de réalisation.

Le nez 636 peut être associé à un organe filiforme 646, permettant notamment de le positionner de façon optimale lors de la mise en place. Cet organe filiforme peut également assurer le retrait du nez, une fois la cage mise en place. En vue de l'introduction, on fixe tout d'abord les deux branches 654 et 655 par rapport au nez 636, par exemple par vissage. On notera que, dans ce mode de réalisation, les branches n'ont pas à s'étendre à la périphérie du corps de cage 601, à savoir qu'elles peuvent être médianes, par rapport à ce dernier.

Puis, on rapporte un pied 610 pourvu d'une traverse 611, afin de solidariser entre elles les deux branches, en vue de former une fourche. On introduit alors le corps de cage dans l'espace intervertébral, comme décrit ci-dessus, en utilisant le nez 636 ainsi que, le cas échéant, l'organe filiforme 646 en vue d'un meilleur positionnement du nez.

Une fois le corps de cage mis en place, on retire les deux branches par rapport au nez, puis on enlève le nez proprement dit grâce à l'organe filiforme 646. Ce retrait du nez peut être opéré, soit par l'extérieur du corps de cage 601, comme sur la figure 31, soit par l'intérieur de ce dernier comme sur la figure 32.

De plus, comme illustré aux figures 33 et 34, on peut prévoir de réaliser le nez 736 en différents éléments 736₁, 736₂ et 736₃. Ces éléments, qui peuvent être assemblés les uns avec les autres au moment de la pose, par tout moyen approprié, sont solidaires de trois branches 754 à 756 de l'outil de pose. Une fois la cage mise en place, il est possible de désassembler ces trois éléments, puis de les retirer par l'intérieur creux du corps de cage 701, comme montré à la figure 34.

A titre de variante supplémentaire, le nez 736 peut être formé d'un nombre d'éléments, différent de trois. De plus, la forme géométrique de ces éléments peut être différente de celle décrite sur cette figure.

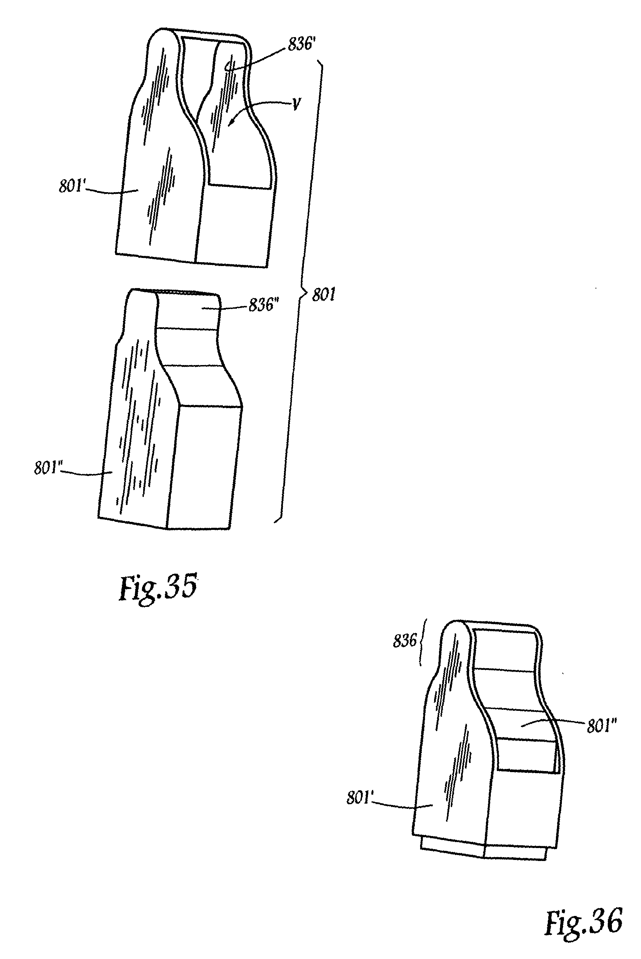

Les figures 35 et suivantes illustrent une variante avantageuse de l'invention, dans laquelle le corps de cage 801 est formé d'une enveloppe 801', ainsi que d'au moins un premier insert 801 ". De façon plus précise, le ou chaque premier insert est destiné à coopérer avec l'enveloppe, au moment de la pose, alors qu'il est prévu un insert supplémentaire 803 destiné à coopérer avec l'enveloppe après la pose, et après avoir retiré le premier insert hors du volume intérieur de l'enveloppe.

L'enveloppe présente une partie principale, terminée par une saillie 836' destinée à former un nez 836, selon l'un ou l'autre des modes de réalisation précédents. Cette enveloppe définit un volume intérieur V, en étant formée par exemple par une paroi mince d'un matériau approprié, notamment un métal ou un matériau plastique biocompatible.

Le premier insert présente la même forme que l'enveloppe, et en particulier, une saillie 836" analogue celle 836'. Lorsqu'il a été introduit dans son volume intérieur, cet insert 801' ne fait pas saillie par rapport à l'avant de cette enveloppe (figure 36). Dans ces conditions, l'ensemble formé par les saillies 836' et 836" de l'enveloppe et du premier insert définissent un nez d'introduction 836, au sens de l'invention, comme le montre la figure 36. Cette enveloppe et ce premier insert sont alors introduits dans l'espace intervertébral, selon une des méthodes décrites ci-dessus.

Une fois cette introduction réalisée, il s'agit de retirer le premier insert hors du volume intérieur de l'enveloppe, tout laissant cette dernière entre les vertèbres. On notera que le matériau constitutif de cette enveloppe doit avoir une résistance suffisante, afin de ne pas s'écraser lors de l'opération, une fois le premier insert enlevé. On notera également que cette résistance n'a pas besoin d'être extrêmement élevée, puisque l'effort exercé par les vertèbres du patient endormi est relativement faible. De plus, comme on le verra dans ce qui suit, il est avantageux que l'enveloppe puisse s'écraser si on exerce une force plus importante.

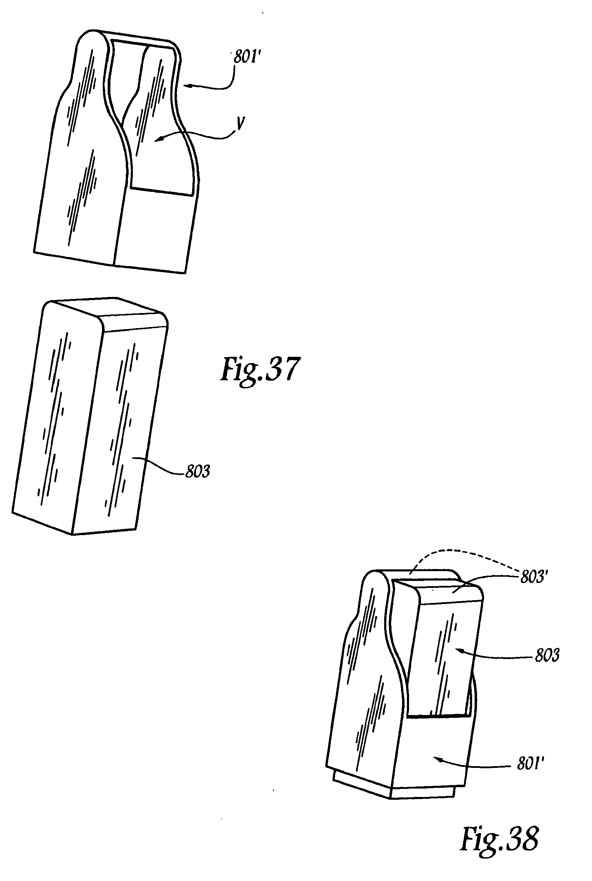

Puis comme illustré aux figures 37 et 38, on introduit l'insert supplémentaire 803 dans le volume intérieur de l'enveloppe 801'. A cet égard, on notera que ce second insert présente des dimensions plus grandes que cette enveloppe, et en particulier, qu'il fait saillie hors de l'enveloppe par deux bourrelets latéraux 803'. On notera que l'ensemble formé par ce second insert et l'enveloppe présente non plus une forme asymétrique avec un nez, mais une forme symétrique de type parallélépipède. Ce mode de réalisation est avantageux dans la mesure où il permet, d'une part, d'assurer une introduction aisée du corps de cage grâce à l'emploi « extemporané» d'un nez et, d'autre part, d'utiliser une forme de cage habituelle, moyennant remplacement du premier insert pourvu du nez par le second insert de forme massive.

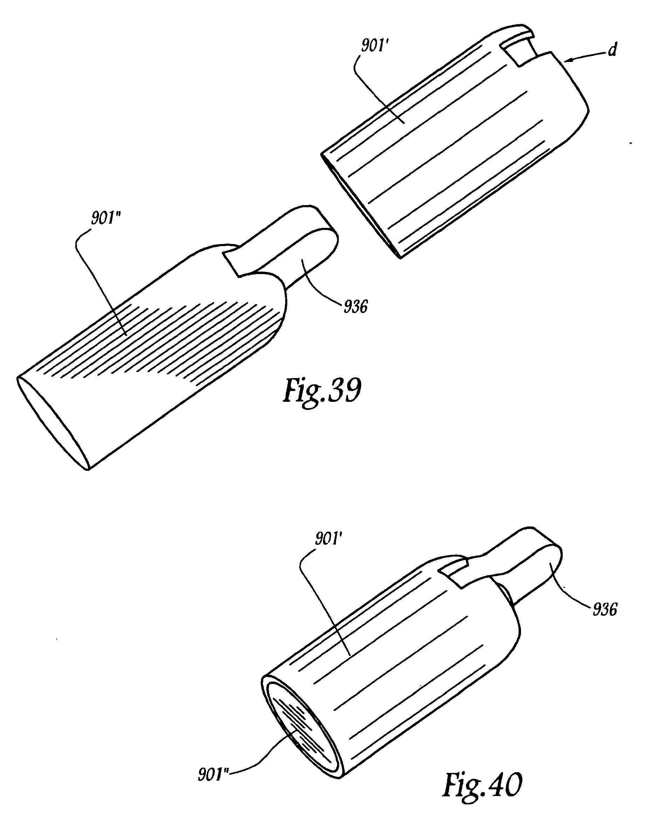

Les figures 39 et suivantes illustrent une variante supplémentaire de réalisation, faisant intervenir un premier insert 901" destiné à la pose, ainsi que plusieurs inserts supplémentaires 903 et 905, destinés à remplacer ce premier insert après la mise en place.

Contrairement au précédent mode de réalisation, l'enveloppe 901' est sensiblement cylindrique, avec une partie antérieure en forme de tronçon de sphère, de façon à former un débouché d de diamètre inférieur à celui du cylindre. Par ailleurs, l'insert de pose 901" comprend tout d'abord une partie principale, de même forme que les parois de l'enveloppe, qui est terminée par un embout 936, formant nez excentré au sens de l'invention (voir figures 39 et 40).

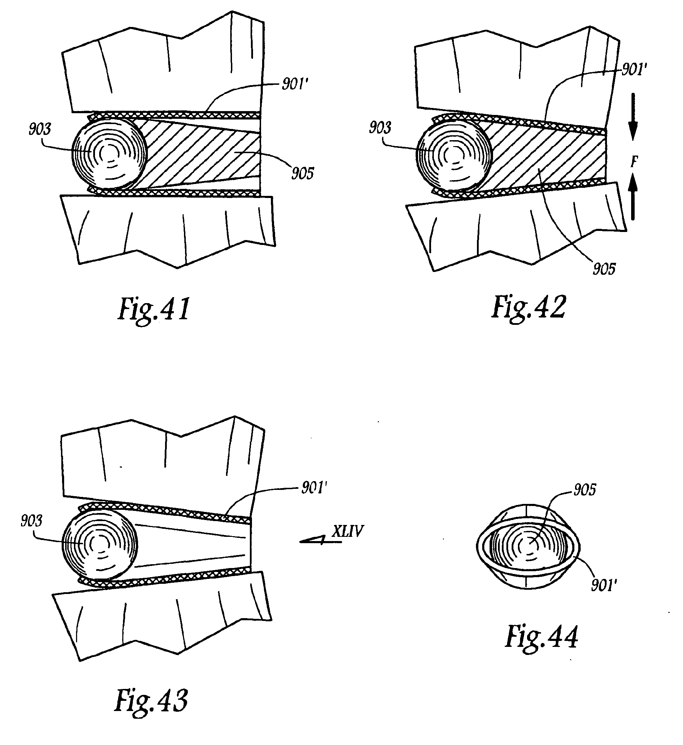

L'ensemble formé par cette enveloppe et ce premier insert peut alors être mis en place entre les vertèbres, selon une des méthodes décrites ci-dessus. Puis, on retire ce premier insert 901 ", que l'on remplace en l'occurrence par deux inserts supplémentaires 903 et 905 (voir figures 41). De façon plus précise, on introduit tout d'abord une sphère 903, que l'on fait déboucher à la partie antérieure de l'enveloppe. Cette sphère permet ainsi d'éviter l'effondrement antérieur de l'enveloppe. De plus, on peut introduire dans la partie postérieure de l'enveloppe un troisième insert 905, dont la hauteur diminue en l'occurrence vers l'arrière.

Une fois que ces deux inserts supplémentaires ont été placés dans le volume intérieur de l'enveloppe, on peut appliquer une force postérieure F, tendant à rapprocher les deux vertèbres l'une de l'autre (figure 42). Cette force peut être initiée par le patient lui-même, une fois réveillé, ou bien par la mise en place d'un hauban postérieur. Dans ces conditions, ceci a tendance à plaquer les parois postérieures de l'enveloppe contre les parois en regard du troisième insert 905 dont on a vu qu'elles sont effilées vers l'arrière.

Les figures 43 et 44 illustrent un mode de réalisation alternatif de celui des figures précédentes. On retrouve là encore la sphère antérieure 903 sensiblement rigide, assurant une fonction d'anti-effondrement, mais en revanche pas de troisième insert. L'enveloppe 901 est également déformable, en fonction de la force arrière susceptible d'être appliquée par un hauban ou par le patient lui-même.

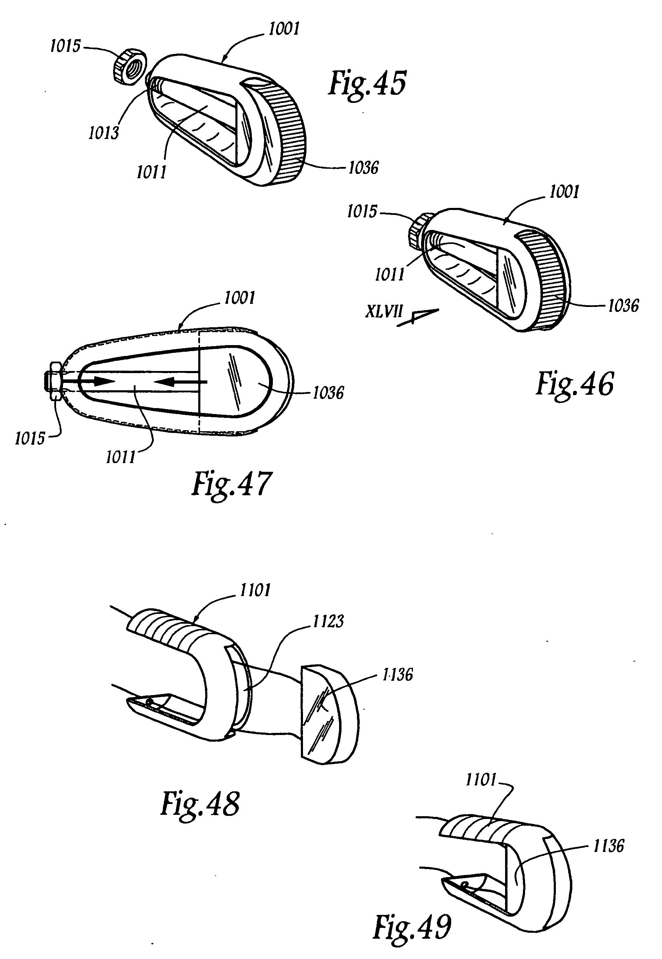

Les figures 45 à 47 illustrent une variante supplémentaire de l'invention, dans laquelle le corps de cage comprend une enveloppe 1001 en forme de ruban, telle que décrite par exemple dans FR-A-2 913 328, au nom du présent Demandeur. Ce ruban est associé à une tige 1011 qui le traverse d'arrière en avant, laquelle est pourvue d'un filetage 1013 coopérant avec un écrou 1015, dans la partie arrière de ce ruban. De plus, cette tige est prolongée par un embout 1036 susceptible de former le nez d'introduction au sens de l'invention. De façon plus précise, la partie antérieure du ruban est creusée d'une échancrure, au travers de laquelle peut coulisser cet embout.

Dans la position d'introduction illustrée sur la figure 45, l'embout 1036 fait largement saillie au-delà de l'échancrure, de façon à assurer sa fonction de nez de d'introduction. Puis, une fois cette mise en place réalisée, le chirurgien manipule la tige filetée, de manière à escamoter l'embout dans la partie antérieure du ruban, comme illustré aux figures 46 et 47. Le chirurgien ajuste alors l'écrou, de façon à mettre en tension la tige longitudinale. Ce mode de réalisation est particulièrement avantageux puisque, dans la première position de la figure 45, l'embout assure la fonction de nez et, dans la position rétractée des figures 46 et 47, il assure une fonction d'anti-effondrement, alors que la tige forme hauban longitudinal.

Les figures 48 et 49 illustrent une variante supplémentaire de réalisation de l'invention. Dans cette variante, on retrouve une enveloppe 1101 en forme de C, telle que par exemple décrite dans FR-A-2 913 328 au nom du présent Demandeur. Cette enveloppe en forme de C est creusée, dans sa partie antérieure, d'une encoche 1123 permettant le passage d'un nez. De façon plus précise, ce nez est formé par un embout 1136 susceptible d'être rapporté contre les parois de l'enveloppe en vue de sa mise en place entre les vertèbres.

Cet embout peut faire saillie au travers de l'encoche, de façon à former le nez d'introduction. Puis, après la mise en place opérée de façon classique, cet embout peut être escamoté afin d'éviter l'effondrement de l'enveloppe, comme dans le mode de réalisation précédent (figure 49). On peut avantageusement déplacer cet embout au moyen de fils 1146 et prévoir, en outre, d'utiliser une tige de mise en tension longitudinale non représentée, comme précédemment.

L'enveloppe en C des figures 48 et 49 peut être associée à un cache utilisé pendant la pose, tel celui 350 des figures 20 et 21, permettant de noyer les arêtes vives du C afin de préserver la racine nerveuse. Ce cache, qui coopère avec le corps de cage pendant l'introduction, peut également intégrer, de façon avantageuse, le nez d'introduction 1136. Cependant, deux organes distincts peuvent également remplir ces fonctions respectives, de cache de protection, et de nez d'introduction.

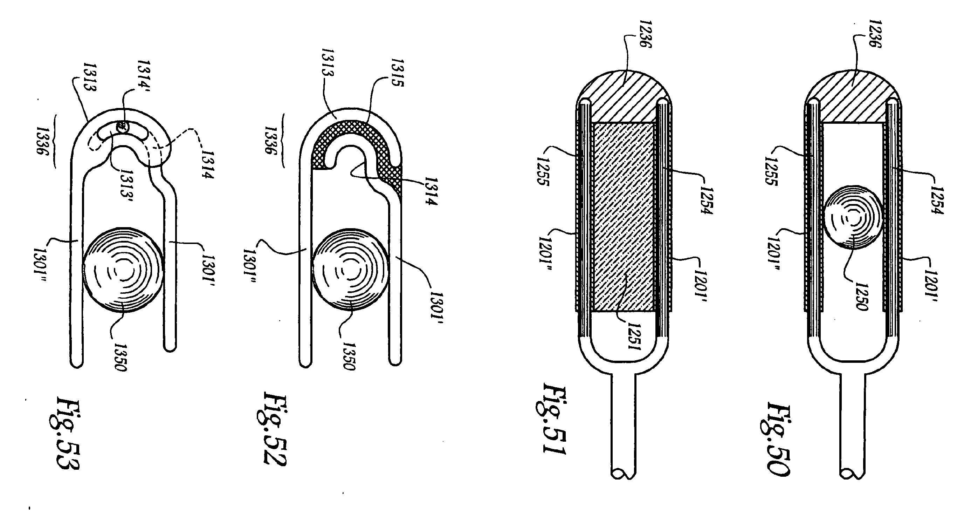

Les figures 50 et 51 illustrent deux modes de réalisation supplémentaires. On retrouve un corps de cage qui est formé de deux plateaux rigides 1201' et 1201" destinés à reposer contre deux vertèbres correspondantes, ainsi que d'un corps intercalaire. Ce dernier, qui autorise un déplacement mutuel entre les deux plateaux rigides, peut être une sphère 1250 (figure 50) ou un bloc massif 1251 (figure 51).

On utilise une fourche de mise en place, dont les branches 1254 et 1255 s'étendent longitudinalement à travers les deux plateaux. De plus, un nez 1236 coopère avec ces fourches, comme par exemple dans le mode de réalisation des figures 30 à 32. On notera que, lorsque ces fourches pénètrent dans les plateaux, ceci permet non seulement de mettre le nez en place, mais également de maintenir les plateaux l'un par rapport à l'autre, en vue d'une introduction aisée entre les vertèbres. Une fois le corps de cage mis en place entre ces vertèbres, on retire alors les branches de la fourche, ainsi qu'éventuellement le nez comme décrit l'un ou l'autre des modes de réalisations précédents.

Les figures 52 et 53 illustrent un autre mode de réalisation, dans lequel les deux plateaux 1301' et 1301" coopèrent mutuellement, afin de former le nez d'introduction 1336. De façon plus précise, le plateau inférieur possède une branche recourbée 1313 en arc de cercle, qui s'étend vers le plateau supérieur, alors que ce dernier présente une saillie en forme de crochet 1314, s'étendant au voisinage de cette branche en arc de cercle. Dans ces conditions, le nez est formé par cette branche et ce crochet, lesquels s'étendent sur une faible largeur de la partie avant des plateaux, en vue de face, en étant en outre décalés axialement de façon à constituer un nez excentré.

De plus, à la figure 52, un matériau élastomère 1315 est avantageusement interposé entre la branche et le crochet, étant entendu qu'il est cependant optionnel. Dans ce mode de réalisation, le nez assure, outre sa fonction classique d'introduction, une fonction supplémentaire de guidage du mouvement. A cet égard, on notera que la branche et le crochet assurent une butée, à la fois en extension et en flexion, des deux plateaux l'un par rapport à l'autre. Ceci permet de former un guide pour le couplage du mouvement vertébral, tout en assurant en outre une possibilité de translation avant/arrière entre les deux plateaux.

A la figure 53, le crochet 1314 est pourvu d'un pion 1314' susceptible de coulisser le long d'une lumière 1313' ménagée dans la branche 1313. Dans ces conditions, le mouvement relatif de plateau est imposé, par déplacement du pion le long de cette lumière, et non plus guidé comme sur la figure 52.

Parmi les agencements des figures 50 à 53, seul celui de la figure 51 appartient à l'invention, en faisant appel à un bloc intercalaire relativement peu souple. En revanche, les agencements utilisant une sphère sont de type prothèse.

Dans les différents modes de réalisation présentés ci-dessus, on a décrit des corps de cage destinés à être introduits à l'intérieur de l'espace intervertébral. On peut les associer à au moins un élément postérieur extra-discal, reliant deux vertèbres, en particulier deux vertèbres adjacentes. Un tel élément extra-discal est propre à limiter, voire à empêcher, les mouvements de flexion et/ou d'extension intervertébrale, afin de rester dans le domaine de l'arthrodèse.

De tels éléments extra-discaux, de type postérieur, peuvent être réalisés sous toute forme appropriée. On citera par exemple l'utilisation de plaques, ou encore de dispositifs plus perfectionnés tels que ceux décrits dans l'une ou l'autre des demandes de brevet français suivantes au nom du Demandeur :
07 05 371 du 24 juillet 2007,
08 02 508 du 6 mai 2008, et
08 55 574 du 14 août 2008.

De façon avantageuse, ces éléments extra-discaux sont segmentés, à savoir qu'ils relient deux vertèbres adjacentes. Egalement de façon avantageuse, ils sont articulés sur les deux vis, notamment de type pédiculaire, qu'ils relient. Enfin, ces éléments extra-discaux sont mis en tension, de manière à constituer un hauban ou un étai, étant entendu qu'il était possible d'associer deux éléments distincts formant respectivement hauban et étai.

Selon une variante avantageuse de l'invention non représentée, on peut équiper des corps de cage existants au moyen d'éléments rapportés, destinés à former le nez d'introduction. Ces éléments rapportés sont ainsi, en quelque sorte, des capuchons que l'on vient adapter sur la face antérieure du corps de cage classique. Un tel capuchon peut être posé contre le corps de cage, ou bien être lié de manière plus ferme avec ce corps de cage. Dans cette dernière possibilité, on peut prévoir que le capuchon possède une zone de fixation, relativement souple, permettant de coiffer le corps de cage, ainsi qu'une zone terminale rigide, formant le nez.

Dans les différents modes de réalisation, décrits ci-dessus comme appartenant à l'invention, on utilise des organes mécaniques adaptés pour assurer une arthrodèse, telle que définie au début de la présente description. L'homme du métier affectera donc à ces organes les caractéristiques appropriées, en particulier en termes de raideur, tout en prenant en compte la nature de leur combinaison avec l'élément postérieur éventuel.

## Revendications

1. Ensemble de stabilisation intervertébrale pour arthrodèse, comprenant un corps de cage (1 ; 1' ; 1" ; 101 ; 201 ; 301 ; 401 ; 401'), destiné à être introduit par impaction dans l'espace intervertébral (E), cet ensemble comprenant en outre un nez (36 ; 36' ; 36" ; 136, 137 ; 236 ; 336 ; 436, 437) d'introduction du corps de cage dans l'espace intervertébral, de sorte que, quand on introduit ce nez dans l'espace intervertébral (E). la mise en rotation de ce nez induit une ouverture de cet espace intervertébral et, de façon simultanée, un déplacement du corps de cage à la façon d'une came **caracterisé en ce que** le nez est solidaire ou propre à être solidarisé avec ce corps de cage, dans une position excentrée.

2. Ensemble selon la revendication 1, **caractérisé en ce que**, vus de dessus, le corps de cage (1 : 1 ' ; 1" ; 101) et le nez (36 ; 36' ; 36" ; 136. 137) présentent des axes principaux respectifs (A, A'), qui sont distincts.

3. Ensemble selon la revendication 2, **caractérisé en ce que** les axes principaux (A, A') du nez et du corps de cage sont parallèles.

4. Ensemble selon l'une quelconque des revendications précédentes, **caractérisé en ce que** le corps de cage (1 ; 1' ; 1" ; 101) présente un bord extérieur arrondi (4 ; 4' ; 4" ; 104), à l'opposé du nez (36 ;36' ;36" ;136) par rapport à un plan médian (P) de ce corps.

5. Ensemble selon l'une quelconque des revendications précédentes, **caractérisé en ce que**, vu de côté, les dimensions (h, H) du corps de cage diminuent vers l'arrière.

6. Ensemble selon la revendication 5, **caractérisé en ce que** les parois extérieures (2, 3) du corps de cage, destinées à entrer en contact avec les corps vertébraux, définissent un angle (a + α') de mise en lordose supérieur à 10°, notamment voisin de 15°.

7. Ensemble selon l'une quelconque des revendications précédentes, **caractérisé en ce que**, au moins dans une position d'introduction, le nez (36 ; 36' ; 36" ; 136, 137) fait saillie vers l'avant, depuis une face avant (12) du corps de cage ,

8. Ensemble selon l'une quelconque des revendications précédentes, **caractérisé en ce que**, vu de côté, le nez présente la même hauteur (H) que la partie la plus haute du corps de cage.

9. Ensemble selon l'une des revendications précédentes, **caractérisé en ce que** le corps de cage comprend une enveloppe (801' ; 901') définissant un volume intérieur, et **en ce qu'**il est prévu un premier insert (801" ; 901 "), ou insert de pose, propre à être rapporté dans le volume intérieur de l'enveloppe de façon à former le nez (836 ; 936).

10. Ensemble de stabilisation selon la revendication précédente, **caractérisé en ce que** l'insert de pose est propre à être enlevé hors du volume intérieur de l'enveloppe, et **en ce qu'**il est prévu au moins un insert supplémentaire (803 ; 903, 905), susceptible d'être introduit dans le volume intérieur de l'enveloppe, à la place de l'insert de pose.

11. Ensemble selon la revendication 9 ou 10, **caractérisé en ce que** l'enveloppe (801' ; 901') est susceptible d'être déformée, notamment en vue d'adopter une configuration lordosée.

12. Ensemble selon l'une des revendications précédentes, **caractérisé en ce que** le nez (1036 ; 1136) est susceptible d'adopter au moins une position en saillie par rapport au corps de cage (1001 ; 1101), dans laquelle il assure l'introduction de ce dernier, ainsi qu'une position escamotée à l'intérieur du corps de cage, dans laquelle il assure une fonction d'anti-effondrement antérieur.

13. Ensemble selon la revendication précédente, **caractérisé en ce que** le nez escamotable est associé à une tige de réglage (1011), laquelle assure une fonction de haubanage longitudinal dans la position escamotée du nez.

14. Ensemble selon l'une des revendications 1 à 8, **caractérisé en ce que** le nez (36") est réalisé monobloc avec le corps de cage (1 ").

15. Ensemble selon l'une des revendications 1 à 8, **caractérisé en ce que** le nez (36) est formé dans un insert (30), propre à être rapporté de manière amovible par rapport au corps de cage (1).

16. Ensemble selon la revendication précédente, **caractérisé en ce que** le corps de cage (1) est creusé d'un logement (14), dans lequel est reçue une embase (32), à partir de laquelle s'étend le nez (36).

17. Ensemble selon la revendication précédente, **caractérisé en ce que** les parois du logement (14) et de l'embase (32) sont telles qu'elles empêchent toute rotation mutuelle, en présentant notamment une forme polygonale, notamment carrée.

18. Ensemble selon la revendication 16 ou 17, **caractérisé en ce que** l'embase (32) est taraudée.

19. Ensemble selon l'une des revendications 15 à 18, **caractérisé en ce que** le nez (636 ; 736) est propre à être fixé, de façon amovible, à au moins deux branches (654, 655 ; 754 - 756) d'un outil de pose.

20. Ensemble selon la revendication précédente, **caractérisé en ce que** le nez (736) est formé de plusieurs éléments (736₁ - 736₃), ces différents éléments étant susceptibles d'être assemblés les uns avec les autres lors de l'introduction, alors que ces différents éléments peuvent être désolidarisés les uns par rapport aux autres après l'introduction, de manière à être retiré, notamment via le volume intérieur du corps de cage (701).

21. Ensemble selon l'une des revendications 15 à 20, **caractérisé en ce que** le nez est réalisé en un matériau permettant la greffe osseuse.

22. Ensemble selon l'une des revendications 1 à 8, **caractérisé en ce que** l'ensemble de stabilisation comprend en outre un organe de pose (150 ; 450) du corps de cage (101 ; 401 ; 401'), et le nez (136, 137 ; 436, 437) est formé par une partie de cet organe de pose, dans une position de solidarisation entre cet organe et ce corps de cage.

23. Ensemble selon la revendication 22, **caractérisé en ce que** le corps de cage (101 ; 401) est creusé de moyens de passage excentrés (108, 109 ; 408, 409 ; 408', 409'), alors que l'organe de pose (150 ; 450) comprend deux branches (154, 155 ; 454, 455) propres à pénétrer dans ces moyens de passage, ces branches étant terminées par deux embouts (136, 137 ; 436, 437) propres à faire saillie vers l'avant de ces moyens de passage, de manière à former le nez.

24. Ensemble selon la revendication 23, **caractérisé en ce que** chaque embout (436, 437) s'étend dans le prolongement d'une branche respective (454, 455).

25. Ensemble selon la revendication 23 ou 24, **caractérisé en ce que** l'organe de pose (150) comprend en outre un piston (159), propre à repousser le corps de cage (101) à l'opposé des branches (154, 155).

26. Ensemble selon la revendication 23 ou 24, **caractérisé en ce que** l'organe de pose (450) comprend une tige filetée (459), propre à pénétrer dans un trou taraudé (411) ménagé dans le corps de cage.

27. Ensemble selon l'une des revendications 23 à 26, **caractérisé en ce que** les moyens de passage sont des rainures (108, 109; 408, 409) creusées dans le corps de cage.

28. Ensemble selon l'une quelconque des revendications 23 à 26, **caractérisé en ce que** les moyens de passage sont des ouvertures excentrées (408', 409') débouchant sur les faces avant et arrière du corps de cage (401') mais ne débouchant pas sur les faces latérales du corps de cage.

29. Ensemble selon l'une des revendications 1 à 28, comprenant en outre au moins un élément postérieur extra-discal, propre à limiter, voire à empêcher, la flexion et/ou l'extension intervertébrale.

30. Ensemble selon l'une des revendications 1 à 29, **caractérisé en ce qu'**il est prévu un cache (350), permettant de cacher les arêtes vives du corps de cage (301) pendant l'introduction.

31. Ensemble selon la revendication précédente, **caractérisé en ce que** le cache est pourvu du nez d'introduction.

32. Ensemble de stabilisation selon l'une des revendications 1 à 31, **caractérisé en ce que** le corps de cage comprend deux plateaux (1201' ; 1201 ") destinés à reposer contre les vertèbres, ainsi qu'un élément intermédiaire (1251) propre à assurer un débattement relatif entre ces plateaux, le nez étant solidaire d'un outil de pose, comportant deux branches (1254, 1255) s'étendant dans ces deux plateaux.

33. Ancillaire de pose de l'ensemble de stabilisation selon l'une des revendications 15 à 21, comprenant un organe de préhension (52 ; 52') par un chirurgien, des moyens de blocage (58 ; 58') du corps de cage (1 ; 1') par rapport à l'organe de préhension, **caractérisé en ce que** l'ancillaire comprend aussi des moyens (56 ; 56') d'immobilisation temporaire du nez (36 ; 36') par rapport au corps de cage (1 ; 1').

34. Ancillaire selon la revendication 33, **caractérisé en ce que** les moyens de blocage comprennent au moins une nervure (58) de l'organe de préhension (52), propre à coopérer avec une rainure ménagée par le corps de cage (1).

35. Ancillaire selon la revendication 33, **caractérisé en ce que** les moyens de blocage comprennent au moins deux doigts (58') de l'organe de préhension (52'), propres à pénétrer dans au moins deux orifices ménagés dans le corps de cage (1').

36. Ancillaire selon l'une des revendications 33 à 35, **caractérisé en ce que** les moyens d'immobilisation comprennent une tige filetée (54) de l'organe de préhension (52), propre à pénétrer dans un orifice taraudé (34) de l'insert (30) pourvu du nez (36).

## Claims

1. An intervertebral stabilization assembly for arthrodesis, comprising a cage body (1; 1'; 1"; 101; 201; 301; 401; 401'), intended to be introduced by impaction into the intervertebral space (E), this assembly further comprising a nose (36; 36'; 36"; 136, 137; 236; 336; 436, 437) for introducing the cage body into the intervertebral space, so that when this nose is introduced into the intervertebral space (E), the rotating of this nose causes this intervertebral space to open up and, at the same time, causes the cage body to move in the manner of a cam, **characterized in that** the nose is secured to or able to be secured to this cage body, in an eccentric position.

2. The assembly as claimed in claim 1, **characterized in that**, when viewed from above, the cage body (1; 1'; 1"; 101) and the nose (36; 36'; 36"; 136, 137) have respective main axes (A, A') which are separate.

3. The assembly as claimed in claim 2, **characterized in that** the main axes (A, A') of the nose and of the cage body are parallel.

4. The assembly as claimed in any one of the preceding claims, **characterized in that** the cage body (1; 1'; 1"; 101) has a rounded exterior edge (4; 4'; 4"; 104), on the opposite side from the nose (36; 36'; 36"; 136) with respect to a mid-plane (P) of this body.

5. The assembly as claimed in any one of the preceding claims, **characterized in that**, when viewed from the side, the dimensions (h, H) of the cage body decrease toward the rear.

6. The assembly as claimed in claim 5, **characterized in that** the exterior walls (2, 3) of the cage body, which are intended to come into contact with the vertebral bodies, define a lordosis-inducing angle (α + α') greater than 10°, notably close to 15°.

7. The assembly as claimed in any one of the preceding claims, **characterized in that**, in at least one insertion position, the nose (36; 36'; 36"; 136, 137) projects forward, from a front face (12) of the cage body.

8. The assembly as claimed in any one of the preceding claims, **characterized in that**, when viewed from the side, the nose has the same height (H) as the tallest part of the cage body.

9. The assembly as claimed in one of the preceding claims, **characterized in that** the cage body comprises an envelope (801'; 901') defining an interior volume, and **in that** a first insert (801"; 901"), or implanting insert, is provided, this insert being able to be attached inside the interior volume of the envelope so as to form the nose (836; 936).

10. The stabilization assembly as claimed in the preceding claim, **characterized in that** the implanting insert can be removed from the interior volume of the envelope, and **in that** at least one additional insert (803; 903, 905) is provided, the latter being able to be introduced into the interior volume of the envelope in place of the implanting insert.

11. The assembly as claimed in claim 9 or 10, **characterized in that** the envelope (801'; 901') can be reformed, notably with a view to adopting a lordosed configuration.

12. The assembly as claimed in one of the preceding claims, **characterized in that** the nose (1036; 1136) is able to adopt at least one position protruding in relation to the cage body (1001; 1101), in which position it allows this cage body to be inserted, and a position retracted inside the cage body, in which position it performs an anterior anti-collapse function.

13. The assembly as claimed in the preceding claim, **characterized in that** the retractable nose is associated with an adjusting rod (1011) which performs a longitudinal bracing function when the nose is in the retracted position.

14. The assembly as claimed in one of claims 1 to 8, **characterized in that** the nose (36") is produced as one piece with the cage body (1").

15. The assembly as claimed in one of claims 1 to 8, **characterized in that** the nose (36) is formed in an insert (30) able to be attached removably in relation to the cage body (1).

16. The assembly as claimed in the preceding claim, **characterized in that** the cage body (1) has hollowed into it a housing (14) in which there is housed a mount (32) from which the nose (36) extends.

17. The assembly as claimed in the preceding claim, **characterized in that** the walls of the housing (14) and of the mount (32) are such that they prevent any mutual rotation, notably having a polygonal, notably square, shape.

18. The assembly as claimed in claim 16 or 17, **characterized in that** the mount (32) is tapped.

19. The assembly as claimed in one of claims 15 to 18, **characterized in that** the nose (636; 736) can be fixed, removably, to at least two branches (654, 655; 754-756) of an implanting tool.

20. The assembly as claimed in the preceding claim, **characterized in that** the nose (736) is formed of several elements (736₁-736₃), these various elements being able to be assembled with one another at the time of insertion, while these various elements can be detached from one another after insertion, so as to be removed, notably via the interior volume of the cage body (701).

21. The assembly as claimed in one of claims 15 to 20, **characterized in that** the nose is made of a material that allows bone grafting.

22. The assembly as claimed in one of claims 1 to 8, **characterized in that** the stabilization assembly further comprises an implanting member (150; 450) for implanting the cage body (101; 401; 401'), and the nose (136, 137; 436, 437) is formed of part of this implanting member, in a position in which this member and this cage body are secured to one another.

23. The assembly as claimed in claim 22, **characterized in that** the cage body (101; 401) has, hollowed into it, eccentric passage means (108, 109; 408, 409; 408', 409') while the implanting member (150; 450) comprises two branches (154, 155; 454, 455) able to penetrate these passage means, these branches ending in two endpieces (136, 137; 436, 437) able to project toward the front of these passage means, so as to form the nose.

24. The assembly as claimed in claim 23, **characterized in that** each end-piece (436, 437) extends in the continuation of a respective branch (454, 455).

25. The assembly as claimed in claim 23 or 24, **characterized in that** the implanting member (150) further comprises a piston (159) able to push the cage body (101) away from the branches (154, 155).

26. The assembly as claimed in claim 23 or 24, **characterized in that** the implanting g member (450) comprises a threaded rod (459) able to penetrate a tapped hole (411) formed in the cage body.

27. The assembly as claimed in one of claims 23 to 26, **characterized in that** the passage means are grooves (108, 109; 408, 409) hollowed into the cage body.

28. The assembly as claimed in any one of claims 23 to 26, **characterized in that** the passage means are eccentric openings (408', 409') opening onto the front and rear faces of the cage body (401') but not opening onto the lateral faces of the cage body.

29. The assembly as claimed in one of claims 1 to 28, further comprising at least one posterior extra-discal element able to limit, or even prevent, intervertebral flexion and/or extension.

30. The assembly as claimed in one of claims 1 to 29, **characterized in that** a cover (350) is provided to cover the sharp edges of the cage body (301) during insertion.

31. The assembly as claimed in the preceding claim, **characterized in that** the cover is provided with the insertion nose.

32. The stabilization assembly as claimed in one of claims 1 to 31, **characterized in that** the cage body comprises two plates (1201'; 1201") intended to rest against the vertebrae, and an intermediate element (1251) able to provide relative travel between these plates, the nose being secured to an implanting tool comprising two branches (1254, 1255) extending into these two plates.

33. An ancillary for implanting the stabilization assembly as claimed in one of claims 15 to 21, comprising a grasping member (52, 52') for a surgeon to grasp, blocking means (58; 58') for blocking the cage body (1; 1') with respect to the grasping member **characterized in that** the ancillary also includes immobilizing means for immobilizing the nose with respect to the cage body.

34. The ancillary as claimed in claim 33, **characterized in that** the blocking means comprise at least one rib (58) of the grasping member (52), able to collaborate with a groove formed by the cage body (1).

35. The ancillary as claimed in claim 33, **characterized in that** the blocking means comprise at least two fingers (58') of the grasping member (52') which are able to enter at least two orifices formed in the cage body (1').

36. The ancillary as claimed in one of claims 33 to 35, **characterized in that** the immobilizing means comprise a threaded rod (54) of the grasping member (52) able to penetrate a tapped orifice (34) of the insert (30) provided with the nose (36).

## Patentansprüche

1. Wirbelsäulenstabilisierungsanordnung für Arthrodese, welche ein Käfigelement (1; 1'; 1"; 101; 201; 301; 401; 401') umfasst, das dazu bestimmt ist, durch Einschlagen in den Zwischenwirbelraum (E) eingeführt zu werden, wobei diese Anordnung außerdem eine Nase (36; 36'; 36"; 136, 137; 236; 336; 436, 437) zur Einführung des Käfigelements in den Zwischenwirbelraum umfasst, derart, dass, wenn man diese Nase in den Zwischenwirbelraum (E) einführt, das Drehen dieser Nase eine Öffnung dieses Zwischenwirbelraums und gleichzeitig eine Verschiebung des Käfigelements in der Art eines Nockens herbeiführt, **dadurch gekennzeichnet, dass** die Nase mit diesem Käfigelement in einer außermittigen Position fest verbunden oder fest verbindbar ist.

2. Anordnung nach Anspruch 1, **dadurch gekennzeichnet, dass**, von oben gesehen, das Käfigelement (1; 1'; 1"; 101) und die Nase (36; 36'; 36"; 136, 137) jeweilige Hauptachsen (A, A') aufweisen, die voneinander verschieden sind.

3. Anordnung nach Anspruch 2, **dadurch gekennzeichnet, dass** die Hauptachsen (A, A') der Nase und des Käfigelements parallel sind.

4. Anordnung nach einem der vorhergehenden Ansprüche, **dadurch gekennzeichnet, dass** das Käfigelement (1; 1'; 1"; 101) gegenüber der Nase (36; 36'; 36", 136), bezogen auf eine Mittelebene (P) dieses Elements, einen abgerundeten Außenrand (4; 4'; 4"; 104) aufweist.

5. Anordnung nach einem der vorhergehenden Ansprüche, **dadurch gekennzeichnet, dass**, von der Seite gesehen, die Maße (h, H) des Käfigelements nach hinten abnehmen.

6. Anordnung nach Anspruch 5, **dadurch gekennzeichnet, dass** die Außenwände (2, 3) des Käfigelements, die dazu bestimmt sind, mit den Wirbelkörpern in Kontakt zu kommen, einen Winkel (α + α') der Lordosierung definieren, der größer als 10° ist und insbesondere etwa 15° beträgt.

7. Anordnung nach einem der vorhergehenden Ansprüche, **dadurch gekennzeichnet, dass** mindestens in einer Einführungsposition die Nase (36; 36'; 36"; 136, 137) von einer Vorderseite (12) des Käfigelements nach vorn vorsteht.

8. Anordnung nach einem der vorhergehenden Ansprüche, **dadurch gekennzeichnet, dass**, von der Seite gesehen, die Nase dieselbe Hohe (H) wie der höchste Teil des Käfigelements aufweist.

9. Anordnung nach einem der vorhergehenden Ansprüche, **dadurch gekennzeichnet, dass** das Käfigelement eine Hülle (801', 901') umfasst, die ein Innenvolumen definiert, und dadurch, dass ein erster Einssatz (801", 901"), oder Implantierungseinsatz, vorgesehen ist, der dafür eingerichtet ist, in das Innenvolumen der Hülle eingesetzt zu werden, so dass die Nase (836, 936) gebildet wird.

10. Stabilisierungsanordnung nach dem vorhergehenden Anspruch, **dadurch gekennzeichnet, dass** der Implantierungseinsatz dafür eingerichtet ist, aus dem Innenvolumen der Hülle entfernt zu werden, und dadurch, dass mindestens ein zusätzlicher Einsatz (803; 903, 905) vorgesehen ist, der anstelle des Implantierungseinsatzes in das Innenvolumen der Hülle einsetzbar ist.

11. Anordnung nach Anspruch 9 oder 10, **dadurch gekennzeichnet, dass** die Hülle (801'; 901') verformbar ist, insbesondere im Hinblick auf die Einnahme einer lordosierten Konfiguration.

12. Anordnung nach einem der vorhergehenden Ansprüche, **dadurch gekennzeichnet, dass** die Nase (1036; 1136) mindestens eine bezüglich des Käfigelements (1001; 1101) vorstehende Position, in welcher sie die Einführung des Letzteren gewährleistet, sowie eine ins Innere des Käfigelements zurückgezogene Position, in welcher sie eine einem Zusammendrücken auf der anterloren Seite entgegenwirkende Funktion erfüllt, einnehmen kann.

13. Anordnung nach einem der vorhergehenden Ansprüche, **dadurch gekennzeichnet, dass** die einziehbare Nase mit einer Stellstange (1011) gekoppelt ist, welche In der eingezogenen Position der Nase eine Funktion der Längsverspannung erfüllt.

14. Anordnung nach einem der Ansprüche 1 bis 8, **dadurch gekennzeichnet, dass** die Nase (36") mit dem Käfigelement (1") einstückig hergestellt ist.

15. Anordnung nach einem der Ansprüche 1 bis 8, **dadurch gekennzeichnet, dass** die Nase (36) in einem Einsatz (30) ausgebildet ist, der dafür eingerichtet Ist, an dem Käfigelement (1) lösbar angesetzt zu werden.

16. Anordnung nach einem der vorhergehenden Ansprüche, **dadurch gekennzeichnet, dass** in dem Käfigelement (1) eine Aufnahme (14) ausgebildet ist, in welcher ein Grundkörper (32) aufgenommen ist, von dem aus sich die Nase (36) erstreckt.

17. Anordnung nach einem der vorhergehenden Ansprüche, **dadurch gekennzeichnet, dass** die Wände der Aufnahme (14) und des Grundkörpers (32) so beschaffen sind, dass sie jede gegenseitige Verdrehung verhindern, indem sie insbesondere eine polygonale, insbesondere eine quadratische Form aufweisen.

18. Anordnung nach Anspruch 16 oder 17, **dadurch gekennzeichnet, dass** der Grundkörper (32) mit einem Innengewinde versehen ist.

19. Anordnung nach einem der Ansprüche 15 bis 18, **dadurch gekennzeichnet, dass** die Nase (636; 736) dafür eingerichtet ist, an mindestens zwei Schenkeln (654, 655; 754-756) eines Implantationswerkzeugs lösbar befestigt zu werden.

20. Anordnung nach einem der vorhergehenden Ansprüche, **dadurch gekennzeichnet, dass** die Nase (736) von mehreren Elementen (736₁ - 736₃) gebildet wird, wobei diese verschiedenen Elemente bei der Einführung zusammengefügt sein können, während diese verschiedenen Elemente nach der Einführung voneinander gelöst werden können, so dass sie, insbesondere über das Innenvolumen des Käfigelements (701), zurückgezogen werden können.

21. Anordnung nach einem der Ansprüche 15 bis 20, dadurch gekenntzeichnet, dass die Nase aus einem Material hergestellt ist, das die Knochentransplantation ermöglicht.

22. Anordnung nach einem der Ansprüche 1 bis 8, **dadurch gekennzeichnet, dass** die Stabilisierungsanordnung außerdem eine Einrichtung zur Implantation (150; 450) des Käfigelements (101; 401; 401') umfasst und die Nase (136, 137; 436, 437) in einer Befestigungsposition zwischen dieser Einrichtung und diesem Käfigelement von einem Teil dieser Implantatlonseinrichtung gebildet wird.

23. Anordnung nach Anspruch 22, **dadurch gekennzeichnet, dass** das Käfigelement (101; 401) von außermittigen Durchgangsmitteln (108, 109; 408, 409; 408', 409') durchbrochen wird, während die Implantationseinrichtung (150; 450) zwei Schenkel (154, 155; 454, 455) umfasst, die dafür eingerichtet sind, in diese Durchgangsmittel einzudringen, wobei diese Schenkel durch zwei Endstücke (136, 137; 436, 437) abgeschlossen zu werden, die dafür eingerichtet sind, aus diesen Durchgangsmitteln nach vorn herauszuragen, so dass sie die Nase bilden.

24. Anordnung nach Anspruch 23, **dadurch gekennzeichnet, dass** jedes Endstück (436, 437) sich in der Verlängerung eines jeweiligen Schenkels (454, 455) erstreckt.

25. Anordnung nach einem der Ansprüche 23 oder 24, **dadurch gekennzeichnet, dass** die Implantationseinrichtung (150) außerdem einen Kolben (159), der dazu eingerichtet ist, das Käfigelement (101) zurückzuschieben, gegenüber den Schenkeln (154, 155) umfasst.

26. Anordnung nach einem der Ansprüche 23 oder 24, **dadurch gekennzeichnet, dass** die Implantationseinrichtung (450) einen Gewindebolzen (459) umfasst, der dazu eingerichtet ist, in eine Innengewindebohrung (411) einzudringen, die in dem Käfigelement ausgebildet ist.

27. Anordnung nach einem der Ansprüche 23 bis 26, **dadurch gekennzeichnet, dass** die Durchgangsmittel Nuten (108, 109; 408, 409) sind, die in das Käfigelement eingearbeitet sind.

28. Anordnung nach einem der Ansprüche 23 bis 26, **dadurch gekennzeichnet, dass** die Durchgangsmittel außermittige Öffnungen (408', 409') sind, die auf der Vorderseite und der Hinterseite des Käfigelements (401') münden, jedoch nicht auf den lateralen Seiten des Käfigelements münden.

29. Anordnung nach einem der Ansprüche 1 bis 28, welche außerdem mindestens ein außerhalb der Bandscheibe angeordnetes posteriores Element umfasst, das dafür eingerichtet ist, die intervertebrale Biegung und/oder Dehnung zu begrenzen oder sogar zu verhindern.

30. Anordnung nach einem der Ansprüche 1 bis 29, **dadurch gekennzeichnet, dass** eine Abdeckung (350) vorgesehen ist, die es ermöglicht, die scharfen Kanten des Käfigelements (301) während der Einführung zu verdecken.

31. Anordnung nach dem vorhergehenden Anspruch, **dadurch gekennzeichnet, dass** die Abdeckung mit der Einführungsnase versehen ist.

32. Stabilisierungsanordnung nach einem der Ansprüche 1 bis 31, **dadurch gekennzeichnet, dass** das Käfigelement zwei Platten (1201', 1201"'), die dazu bestimmt sind, an den Wirbel zur Anlage zu kommen, sowie ein Zwischenelement (1251), das dafür eingerichtet ist, ein relatives Spiel zwischen diesen Platten sicherzustellen, umfasst, wobei die Nase mit einem Implantationswerkzeug fest verbunden ist, das zwei Schenkel (1254, 1255) aufweist, die sich in diesen zwei Platten erstrecken.

33. Hilfsvorrichtung zur Implantierung der Stabilisierungsanordnung nach einem der Ansprüche 15 bis 21, welche ein Griffstück (52; 52') zum Ergreifen durch einen Chirurgen und Blockiermittel (58; 58') zum Blockieren des Käfigelements (1; 1') bezüglich des Griffstücks umfasst, **dadurch gekennzeichnet, dass** die Hilfsvorrichtung außerdem Fixiermittel (56; 56') zur zeitweiligen Fixierung der Nase (36: 36') bezüglich des Käfigelements (1; 1') umfasst.

34. Hilfsvorrichtung nach Anspruch 33, **dadurch gekennzeichnet, dass** die Blockiermittel mindestens eine Rippe (58) des Griffstücks (52) umfassen, die dafür eingerichtet ist, mit einer Nut zusammenzuwirken, die in dem Käfigelement (1) ausgebildet ist.

35. Hilfsvorrichtung nach Anspruch 33, **dadurch gekennzeichnet, dass** die Blockiermittel mindestens zwei Finger (58') des Griffstücks (52') umfassen, die dafür eingerichtet sind, in mindestens zwei Öffnungen einzudringen, die in dem Käfigelement (1') ausgebildet sind.

36. Hilfsvorrichtung nach einem der Ansprüche 33 bis 35, **dadurch gekennzeichnet, dass** die Fixiermittel einen Gewindebolzen (54) des Griffstücks (52) umfassen, der dafür eingerichtet ist, in eine Innengewindebohrung (34) des Einsatzes (30) einzudringen, der mit der Nase (36) versehen ist.
